# EUROPEAN PATENT APPLICATION

(11) **EP 3 964 532 A1**
(43) Date of publication of application: **09.03.2022**
(21) Application number: 20798650.6
(22) Date of filing: 13.03.2020
(51) Int. Cl.: C07K 19/00, C12N 15/62, C12N 15/63, C12N 5/10, A61K 39/02, A61P 31/04

(54) **FUSION PROTEIN CAPABLE OF SELF-ASSEMBLING INTO PROTEIN NANOPARTICLES AND APPLICATION THEREOF**

(30) Priority: 29.04.2019 CN 201910352203
(71) Applicant: Academy Of Military Medical Sciences, Haidian District Beijing 100850 (CN)
(72) Inventor: WANG, Hengliang, Beijing 100071 (CN); PAN, Chao, Beijing 100071 (CN); ZHU, Li, Beijing 100071 (CN); WU, Jun, Beijing 100071 (CN); HUANG, Jing, Beijing 100071 (CN); ZHANG, Lulu, Beijing 100071 (CN); SUN, Peng, Beijing 100071 (CN); ZENG, Ming, Beijing 102629 (CN); WANG, Bin, Beijing 102629 (CN); LIU, Xiankai, Beijing 100071 (CN); WANG, Dongshu, Beijing 100071 (CN); FENG, Erling, Beijing 100071 (CN); LIU, Bo, Beijing 100071 (CN)
(74) Representative: Isarpatent
(86) International application number: PCT/CN2020/079266
(87) International publication number: WO 2020/220846

(57) **Abstract**

The present invention relates to a recombinant fusion protein and a polysaccharide conjugate vaccine. More specifically, the present invention relates to a fusion protein including a pentamer substrate protein and a peptide sequence capable of forming a trimer, an immunogenic composition including the fusion protein, the application of the fusion protein in immunology, a polynucleotide sequence encoding the fusion protein, an expression vector containing the polynucleotide sequence, and a host cell containing the expression vector.

## Description

### TECHNICAL FIELD

The present invention relates to a fusion protein capable of self-assembling into a protein nanoparticle and application thereof, which belongs to the field of biomedicine.

### BACKGROUND

Vaccines play a vital role in the prevention of pathogenic bacterial infections. The new generation of vaccine products (subunit vaccines) have a more single and clear composition, which makes their products safer. However, these vaccines often have a relatively low immunogenicity, and will be rapidly diluted by body fluids and quickly degraded after being injected into the body. Therefore some adjuvants and suitable delivery systems are often required to promote the maximum immune response. In recent years, extensive research has found that nanoscale vaccines can significantly improve immunological effect. Particularly, nano-carrier vaccines have been continuously used. The main features are that the nano-carrier vaccines can replace adjuvants, and concurrently trigger cellular immunity and humoral immunity, and yield ideal immunological effect especially for relatively weak epitopes, thereby providing a good direction for future vaccine development.

Polysaccharide conjugate vaccines are formed by covalently linking immunogenic polysaccharides to appropriate substrate proteins, and thus the polysaccharide antigens are transformed from T-cell-independent antigens to T-cell-dependent antigens, which enables a long-lasting protective effect in the body. Effective antibodies are produced especially for the elderly, infants and young children. Therefore, polysaccharide conjugate vaccines are considered to be one of the most successful forms of vaccines because of the simultaneous triggering T-cell-dependent and T-cell-independent immune responses. However, polysaccharide conjugate vaccines prepared by the current technology still show weak immune response ability against certain bacteria, which needs to be further improved.

Therefore, on the basis of the current vaccines, if the carrier proteins in the polysaccharide conjugate vaccines can be replaced by protein nanoparticles, the immunological effect will be further improved.

### SUMMARY

The present inventors have carried out a fusion expression of a known carrier protein naturally present in prokaryotes with a peptide fragment capable of forming a trimer, and further used the glycosylation system to link bacterial polysaccharides therein, to successfully obtain nano level glycoproteins. Animal experiments showed that the nano level glycoproteins have significantly improved the antibody level, bactericidal activity and protective effects compared to single polysaccharide and polysaccharide conjugate vaccine with only pentameric protein. This shows that more promising nanoscale polysaccharide conjugate vaccines can be prepared by biological methods, which provides a direction for the next step of the development of polysaccharide conjugate vaccines.

### Fusion protein

Therefore, in the first aspect, the present invention provides a fusion protein capable of self-assembling into a protein nanoparticle. The fusion protein includes an AB5 toxin B subunit (hereinafter also referred to as B5 or B5 protein) capable of forming a pentamer and a trimerization domain sequence (hereinafter also referred to as Tri or Tri sequence) capable of forming a trimer structure.

For convenience, the fusion protein described above is named B5Tri, where B5 is the B subunit in AB5 toxin from bacteria and can form a pentamer. In some embodiments, the B5 protein is selected from cholera toxin B subunit (CTB), Shiga toxin B subunit (StxB), *Escherichia coli* heat-labile enterotoxin B subunit (LTB), and *Bacillus subtilis* toxin B subunit (SubB). In some embodiments, the CTB has an amino acid sequence shown in SEQ ID No. 1, and a nucleotide sequence shown in SEQ ID No. 2; the StxB has an amino acid sequence shown in SEQ ID No. 3, and a nucleotide sequence shown in SEQ ID No. 4; the LTB has an amino acid sequence shown in SEQ ID No. 5, and a nucleotide sequence shown in SEQ ID No. 6; and the SubB has an amino acid sequence shown in SEQ ID No. 7, and a nucleotide sequence shown in SEQ ID No. 8.

AB5 toxin is a common form of microbial toxins in nature, mainly composed of an A subunit and B subunits. These five B subunits form a cyclic pentamer, which is responsible for binding to the surface of a host cell and catalyzing the destruction of the host cell by the A subunit.

CTB is the cholera toxin B subunit, which can specifically bind to the ganglioside GM1 on the surface of most mammals' cells, promote the antigenic protein connected thereto to pass through the mucosal barrier, and at the same time enhance the antigen presentation by dendritic cells (DCs) and other antigen-presenting cells. As such, CTB is a good carrier protein.

StxB is the Shiga toxin B subunit, which possesses powerful immunomodulatory properties, enables identification of exogenous antigens by major histocompatibility complex class I (MHC-I) in antigen-presenting cells, and thus mediates cellular immune response. Therefore, StxB is a very promising non-active and non-toxic vaccine carrier and has been proven to have anti-tumor protective immunity in mice. Furthermore, the ability of StxB to induce cell-mediated immunity also indicates its potential use in the immunoprophylaxis and treatment of infectious diseases.

LTB is the B subunit of *Escherichia coli* heat-labile enterotoxin, having structure and function similar to CTB. Both can promote immune response through the GM1 receptor. In addition, LTB can also bind to some glycolipids or glycoproteins on the cell surface to promote antigen identification and presentation.

SubB is the *Bacillus subtilis* toxin B subunit, similar to cholera toxin and heat-labile enterotoxin. SubB can also trigger immune response, promote T cell activation, and facilitate the release of inflammatory factors.

Tri in the B5Tri fusion protein refers to a polypeptide sequence that can form a trimer structure. In some embodiments, the Tri has an amino acid sequence shown in SEQ ID No. 9, and a nucleotide sequence shown in SEQ ID No. 10. This sequence is artificially designed, has no homology with human protein sequence, and can form a trimer under natural conditions.

The present inventors surprisingly found that pentameric proteins in AB5 toxin such as cholera toxin B subunit (CTB), Shiga toxin B subunit (StxB), *Escherichia coli* heat-labile enterotoxin B subunit (LTB), and *Bacillus subtilis* toxin B subunit (SubB), when subjected to a fusion expression with Tri sequence, can self-assemble into 10-20 nm fusion protein particles. The fusion protein particles each are composed of a pentamer of B5 carrier protein and a trimer of Tri. Due to the characteristics that pentamer and trimer can be formed concurrently, multiple monomeric proteins can spatially self-assemble into a higher polymer, forming 10-20 nm spherical particles.

In some embodiments, the fusion protein contains the AB5 toxin B subunit and the trimerization domain sequence from N terminus to C terminus.

In some exemplary embodiments, the fusion protein contains an amino acid sequence selected from:
(1) an amino acid sequence consisting of amino acid residues at positions 20 to 172 from the N terminus of the sequence shown in SEQ ID NO. 21;
(2) an amino acid sequence consisting of amino acid residues at positions 20 to 157 from the N terminus of the sequence shown in SEQ ID NO. 23;
(3) an amino acid sequence consisting of amino acid residues at positions 20 to 172 from the N terminus of the sequence shown in SEQ ID NO. 25; and
(4) an amino acid sequence consisting of amino acid residues at positions 20 to 209 from the N terminus of the sequence shown in SEQ ID NO. 27.

Preparing polysaccharide conjugate vaccines by biological methods mainly relies on the relaxed substrate specificity of glycosyltransferases, where the glycosyltransferases, bacterial surface polysaccharides and substrate proteins are subjected to a co-expression in bacteria (generally *Escherichia coli),* so that the polysaccharides are transferred to the substrate proteins under the catalysis of the glycosyltransferases, and the glycoproteins can be obtained after one step of purification. Specifically, the glycosyltransferase PglL of *Neisseria meningitidis* has been widely used because it has relaxed substrate specificity and can recognize almost all glycans. Therefore, the fusion protein of the present invention can be further fused with a sequence containing glycosylation sites, as a result, by co-expression with glycosyltransferases, bacterial polysaccharides can be linked to the fusion protein of the present invention to prepare nanoscale polysaccharide conjugate vaccines.

In this regard, in the second aspect, the present invention also provides a fusion protein, which comprises the fusion protein described in the first aspect and a glycosylation sequence containing an O-glycosylation site. The glycosylation sequence can serve as the substrate protein of the glycosyltransferase (such as PglL) in the O-glycosylation system, and the bacterial O-polysaccharide can be transferred to the glycosylation site contained in the glycosylation sequence under the action of the glycosyltransferase.

In some embodiments, the glycosylation sequence is selected from the peptide fragment containing O-glycosylation site of the pilin protein PilE of *Neisseria meningitidis.* In some embodiments, the O-glycosylation site is the serine at position 63 from the N terminus of PilE. In some embodiments, the peptide fragment containing O-glycosylation site of the pilin protein PilE of *Neisseria meningitidis* includes at least the serine at position 63 from the N terminus of PilE. In some embodiments, the peptide fragment containing O-glycosylation site of the pilin protein PilE of *Neisseria meningitidis* is a peptide fragment containing at least the amino acid residues at positions 45 to 73 from the N terminus.

In some embodiments, the glycosylation sequence contains a peptide fragment consisting of amino acid residues at positions 45 to 73 from the N terminus of PilE.

In some embodiments, the glycosylation sequence contains the sequence shown in SEQ ID NO. 29.

In some embodiments, the fusion protein, from N terminus to C terminus, contains the AB5 toxin B subunit, the trimerization domain sequence, and the glycosylation sequence.

In some exemplary embodiments, the fusion protein contains an amino acid sequence selected from:
(1) an amino acid sequence consisting of amino acid residues at positions 20 to 205 from the N terminus of the sequence shown in SEQ ID NO. 21;
(2) an amino acid sequence consisting of amino acid residues at positions 20 to 190 from the N terminus of the sequence shown in SEQ ID NO. 23;
(3) an amino acid sequence consisting of amino acid residues at positions 20 to 205 from the N terminus of the sequence shown in SEQ ID NO. 25; and
(4) an amino acid sequence consisting of amino acid residues at positions 20 to 242 from the N terminus of the sequence shown in SEQ ID NO. 27.

In some embodiments, any two adjacent domains (e.g., AB5 toxin B subunit and trimerization domain sequence, trimerization domain sequence and glycosylation sequence) contained in the fusion protein described in the first or second aspect are optionally linked by peptide linkers. In some embodiments, the peptide linkers include, but not limited to, KK (lysine-lysine), GP (glycine-proline), GPGP (glycine-proline-glycine-proline), GGS (glycine-glycine-serine), GGGS, GGGGS their repetitions and various combinations thereof. In some embodiments, the peptide linker is a flexible amino acid linker containing glycine (G) and serine (S). In some embodiments, the peptide linker is composed of 1-20 amino acids (e.g., 1-15 amino acids, 1-8 amino acids, 2-6 amino acids, or about 4 amino acids). In some exemplary embodiments, the peptide linker has the sequence shown in SEQ ID NO. 30.

In some embodiments, the fusion protein of the first or second aspect may further include a signal peptide, thereby facilitating a secretory expression of the fusion protein of the present invention in a host cell. In some embodiments, the fusion protein of the present invention contains a signal peptide at its N terminus.

The signal peptide inherently possessed by AB5 toxin B subunit can be integrally fused into the fusion protein of the present invention. Alternatively, the signal peptide can be removed, and replaced with a signal peptide of a protein derived from a prokaryotic cell, such as a signal peptide of a secreted protein (for example, outer membrane protein or periplasmic protein (e.g., DsbA)) from *Escherichia coli.*

In some exemplary embodiments, the signal peptide has the sequence shown in SEQ ID NO. 31.

In some exemplary embodiments, the fusion protein contains an amino acid sequence selected from:
(1) an amino acid sequence consisting of amino acid residues at positions 1 to 205 from the N terminus of the sequence shown in SEQ ID NO. 21;
(2) an amino acid sequence consisting of amino acid residues at positions 1 to 190 from the N terminus of the sequence shown in SEQ ID NO. 23;
(3) an amino acid sequence consisting of amino acid residues at positions 1 to 205 from the N terminus of the sequence shown in SEQ ID NO. 25; and
(4) an amino acid sequence consisting of amino acid residues at positions 1 to 242 from the N terminus of the sequence shown in SEQ ID NO. 27.

In some embodiments, the fusion protein of the first or second aspect may further include a protein tag. Such protein tag is well known in the art, examples of which include, but are not limited to, His, Flag, GST, MBP, HA, Myc, etc., and those skilled in the art know how to select a proper protein tag according to a desired purpose (for example, purification, detection or tracing). In some exemplary embodiments, the fusion protein of the present invention congains a His tag. In some embodiments, the fusion protein of the present invention contains a protein tag at its C terminus.

In some exemplary embodiments, the fusion protein includes the amino acid sequence shown in SEQ ID NO. 21, SEQ ID NO. 23, SEQ ID NO. 25, or SEQ ID NO. 27.

### Protein nanoparticles and preparation thereof

The fusion protein of the present invention can self-assemble into nanoparticles after being expressed in cells. The protein nanoparticles have an average particle size of about 10-20 nm, and each of the protein nanoparticles contains or is composed of a pentamer of B5 protein and a trimer of Tri sequence.

Therefore, in another aspect, the present invention further relates to a protein nanoparticle which is obtained by expressing the fusion protein in a host cell containing a nucleotide sequence encoding the fusion protein of the first aspect.

In another aspect, the present invention provides an isolated nucleic acid molecule which contains a nucleotide sequence encoding the fusion protein of the first aspect.

In another aspect, the present invention provides a vector (such as a cloning vector or an expression vector), which contains the isolated nucleic acid molecule as described above. In some embodiments, the vector is, for example, a plasmid, a cosmid, a bacteriophage, and the like.

In another aspect, the present invention provides a host cell, which contains the isolated nucleic acid molecule or the vector as described above. Such host cells include, but are not limited to, prokaryotic cells such as *E. coli* cells, eukaryotic cells such as yeast cells, insect cells, plant cells, and animal cells (such as mammalian cells, e.g., mouse cells, human cells, etc.). In some embodiments, the host cell is a prokaryotic cell (such as bacterial cells, e.g., *E. coli* cells).

In another aspect, a method for preparing the fusion protein of the first aspect is provided, including culturing the host cell as described above under conditions that allow protein expression, and recovering the fusion protein from the cultured host cell culture. The fusion protein exists in the form of nanoparticles.

In another aspect, a method for preparing the protein nanoparticles is provided, which includes culturing the host cell as described above, under conditions that allow protein expression, and recovering the nanoparticles from the cultured host cell culture.

### Glycoprotein nanoparticles

When the fusion protein of the present invention contains a glycosylation sequence, by co-expression with glycosyltransferases in bacterial cells, the glycosyltransferases can transfer bacterial polysaccharides to the fusion protein of the present invention, thereby obtaining glycoprotein nanoparticles.

Therefore, in the third aspect, the present invention provides a method for preparing glycoprotein nanoparticles, which includes the following steps:
(1) provide bacterial host cells lacking O-antigen ligase, wherein the deletion of endogenous O-antigen ligase can prevent O-antigen polysaccharides from being catalyzed and transferred by O-antigen ligase to lipid A core polysaccharide to form lipopolysaccharides, as a result, free O-antigen polysaccharide can be produced; and
(2) express the fusion protein and the glycosyltransferase PglL described in the second aspect in the bacterial host cell, thereby producing the target glycoprotein nanoparticle.

In some embodiments, the method further includes: (3) purifying the target glycoprotein nanoparticles. In some embodiments, the target glycoprotein nanoparticles are purified by affinity chromatography and/or molecular sieve. In some embodiments, when the fusion protein contains a protein tag, the method may further include the step of detecting the target glycoprotein nanoparticle by using an antibody specific for the protein tag.

In some embodiments, in step (1), the bacterial host cell lacking O-antigen ligase is provided by knocking out the endogenous O-antigen ligase gene in the bacterial host cell. Herein, "knocking out" in the context of a gene means that a host cell carrying the knockout does not produce a functional protein product of the gene. Knockout can be performed in a variety of ways including removing all or part of coding sequences, introducing frameshift mutations so that no functional protein (truncated or nonsense sequence) is produced, removing or changing regulatory components (e.g., promoters) so that no gene transcription occurs, and preventing translation by binding to mRNA, and so on. Generally, knockout is carried out at the level of genomic DNA, so that cell progeny also permanently carry the knockout.

In some embodiments, the O-antigen ligase gene is a *waaL* gene.

In some embodiments, the bacterial host cell is a pathogenic bacterial cell. In some embodiments, the bacterial host cell is a pathogenic gram-negative bacterial (gram-negative pathogenic bacterial) cell. In some embodiments, the bacterial host cell is selected from *Shigella, Escherichia coli, Shigella dysenteriae, Salmonella paratyphi* A, and *Vibrio cholerae.*

In some embodiments, the fusion protein and PglL of the second aspect are co-expressed in the same bacterial host cell. The co-expression is optionally performed by expressing each protein on the same or different expression vectors. The bacterial host cell can be co-transfected with two expression vectors, where the first vector encodes the fusion protein and the second vector encodes the PglL. Alternatively, a single vector that can respectively encode and express the fusion protein and the PglL can be used.

In some embodiments, step (2) includes the following steps:
(2a) introducing a first nucleotide sequence encoding the fusion protein of the second aspect and a second nucleotide sequence encoding the PglL into the bacterial host cell, wherein the first nucleotide sequence and the second nucleoside sequence are provided on the same or different expression vectors (for example, prokaryotic expression vector);
(2b) culturing the bacterial host cell (for example, under a condition that allows protein expression); and
(2c) recovering the target glycoprotein nanoparticles from a cell culture (such as a cell lysate).

In the fourth aspect, the present invention provides a glycoprotein nanoparticle, which includes a protein-polysaccharide conjugate. The conjugate is formed by coupling a bacterial O-polysaccharide and the fusion protein described in the second aspect, and the bacterial O-polysaccharide is coupled to the fusion protein of the second aspect through the O-glycosylation site in the glycosylation sequence of the fusion protein.

In some embodiments, the bacterial O-polysaccharide is selected from pathogenic bacteria O-polysaccharide.

In some embodiments, the bacterial O-antigen polysaccharide is selected from *Shigella* O polysaccharide, *Escherichia coli* 01570 polysaccharide, *Shigella dysenteriae* O polysaccharide, *Salmonella* paratyphi A O polysaccharide, and *Vibrio cholerae* O polysaccharide.

In some embodiments, in the glycoprotein nanoparticle, the AB5 toxin B subunit in the fusion protein exists in a form of pentamer, and the trimerization domain sequence in the fusion protein exists in a form of trimer.

In some embodiments, the average particle size of the glycoprotein nanoparticle is about 25-50 nm.

In some embodiments, the glycoprotein nanoparticle is prepared by the method described in the third aspect.

### Application of glycoprotein nanoparticles

In the fifth aspect, the present invention provides an immunogenic composition or vaccine, including: (i) an immunologically effective dose of the glycoprotein nanoparticle described in the fourth aspect, or (ii) an immunologically effective dose of a mixture including the fusion protein described in the first or second aspect or the protein nanoparticle formed by the fusion protein, and a bacterial polysaccharide (for example, bacterial O-polysaccharide). Preferably, the immunogenic composition or vaccine does not contain an adjuvant.

In some embodiments, the immunogenic composition or vaccine is used to induce an immune response (such as an antibody response) to a bacterial O-polysaccharide in a subject, and/or to prevent and/or treat bacterial infections (such as infections caused by bacteria expressing O-polysaccharides).

In some embodiments, the bacteria are pathogenic bacteria. In some embodiments, the bacteria are gram-negative bacteria. In some embodiments, the bacteria are pathogenic gram-negative bacteria (gram-negative pathogenic bacteria). In some embodiments, the bacteria are selected from *Shigella, Escherichia coli, Shigella dysenteriae, Salmonella paratyphi* A, and *Vibrio cholerae.*

In some embodiments, the bacterial O-polysaccharide is selected from pathogenic bacteria O-polysaccharide. In some embodiments, the bacterial O-antigen polysaccharide is selected from *Shigella* O polysaccharide, *Escherichia coli* 01570 polysaccharide, *Shigella dysenteriae* O polysaccharide, *Salmonella* paratyphi A O polysaccharide, and *Vibrio cholerae* O polysaccharide.

In some embodiments, the immunogenic composition or vaccine further includes a pharmaceutically acceptable carrier and/or excipient.

The glycoprotein nanoparticle, or the immunogenic composition or vaccine of the present invention can be in any form known in the medical field, for example, it can be tablet, pill, suspension, emulsion, solution, gel, capsule, powder, granule, elixir, lozenge, suppository, injection (including injection solutions, freeze-dried powders), inhalant, spray and other forms. A skilled person should understand that a preferred dosage form depends on a desired method of administration and therapeutic use. In some embodiments, the glycoprotein nanoparticle, or the immunogenic composition or vaccine of the present invention is an injection preparation, such as an injection solution, a sterile powder for injection, or a concentrated solution for injection.

The glycoprotein nanoparticle, or the immunogenic composition or vaccine of the present invention can be administered by any suitable method known in the art, including but not limited to oral, rectal, parenteral or topical administration. In some embodiments, the glycoprotein nanoparticle, or the immunogenic composition or vaccine of the present invention is parenterally administered, for example, subcutaneous injection, intravenous injection, intraperitoneal injection, intramuscular injection, intrasternal injection and introduction. The dosage form for parenteral administration may be an injection preparation, including an injection solution, a sterile powder for injection, or a concentrated solution for injection. In addition to active ingredients, the injection preparation may contain a pharmaceutically acceptable carrier such as sterile water, Ringer's solution and isotonic sodium chloride solution. Furthermore, appropriate additives such as an antioxidant, a buffering agent and a bacteriostatic agent can be added based on the properties of drugs.

In addition, other carrier materials and administration methods known in the pharmaceutical field can also be used. The glycoprotein nanoparticle, or the immunogenic composition or vaccine of the present invention can be prepared by any known pharmaceutical process, such as effective preparation and administration method.

In the sixth aspect, the present invention provides a use of the glycoprotein nanoparticle of the fourth aspect or the immunogenic composition or vaccine of the fifth aspect in preparing a product for inducing a specific immune response (for example, an antibody response) to a bacterial O-polysaccharide in a subject and/or for a prevention and/or treatment of bacterial infections (for example, infections caused by bacteria expressing O-polysaccharides). In some embodiments, the product is a vaccine.

In some embodiments, the bacteria are pathogenic bacteria. In some embodiments, the bacteria are gram-negative bacteria. In some embodiments, the bacteria are pathogenic gram-negative bacteria (gram-negative pathogenic bacteria). In some embodiments, the bacteria are selected from *Shigella, Escherichia coli, Shigella dysenteriae, Salmonella paratyphi* A, and *Vibrio cholerae.*

In some embodiments, the bacterial O-polysaccharide is selected from pathogenic bacteria O-polysaccharide. In some embodiments, the bacterial O-antigen polysaccharide is selected from *Shigella* O polysaccharide, *Escherichia coli* 01570 polysaccharide, *Shigella dysenteriae* O polysaccharide, *Salmonella* paratyphi A O polysaccharide, and *Vibrio cholerae* O polysaccharide.

In the seventh aspect, the present invention further provides a method for inducing a specific immune response (such as an antibody response) to bacterial O-polysaccharides in a subject and/or for preventing and/or treating bacterial infections (such as infections caused by bacteria expressing O-polysaccharides), which includes: administering a subject in need with an immunologically effective dose of the glycoprotein nanoparticle according to the fourth aspect or the immunogenic composition or vaccine according to the fifth aspect.

In some embodiments, the bacteria are pathogenic bacteria. In some embodiments, the bacteria are gram-negative bacteria. In some embodiments, the bacteria are pathogenic gram-negative bacteria (gram-negative pathogenic bacteria). In some embodiments, the bacteria are selected from *Shigella, Escherichia coli, Shigella dysenteriae, Salmonella paratyphi* A, and *Vibrio cholerae.*

In some embodiments, the bacterial O-polysaccharide is selected from pathogenic bacteria O-polysaccharide. In some embodiments, the bacterial O-antigen polysaccharide is selected from *Shigella* O polysaccharide, *Escherichia coli* 01570 polysaccharide, *Shigella dysenteriae* O polysaccharide, *Salmonella* paratyphi A O polysaccharide, and *Vibrio cholerae* O polysaccharide.

In some embodiments, the subject is a mammal, such as human.

The present invention further includes the following exemplary embodiments:
Item 1. A fusion protein capable of self-assembling into a protein nanoparticle. The fusion protein includes a bacterium-derived carrier protein B5 capable of forming a pentamer and a polypeptide sequence Tri capable of forming a trimer structure.
Item 2. The fusion protein according to the item 1, where the B5 protein is selected from cholera toxin B subunit (CTB), Shiga toxin B subunit (StxB), *Escherichia coli* heat-labile enterotoxin B subunit (LTB), and *Bacillus subtilis* toxin B subunit (SubB). Preferably, the CTB has an amino acid sequence shown in SEQ ID No. 1, and a nucleotide sequence shown in SEQ ID No. 2; the StxB has an amino acid sequence shown in SEQ ID No. 3, and a nucleotide sequence shown in SEQ ID No. 4; the LTB has an amino acid sequence shown in SEQ ID No. 5, and a nucleotide sequence shown in SEQ ID No. 6; and the SubB has an amino acid sequence shown in SEQ ID No. 7, and a nucleotide sequence shown in SEQ ID No. 8.
Item 3. The fusion protein according to the item 1 or 2, where the Tri is a polypeptide sequence capable of forming a trimer structure. Preferably, the Tri has an amino acid sequence shown in SEQ ID No. 9, and a nucleotide sequence shown in SEQ ID No. 10.
Item 4. The fusion protein according to any one of the items 1-3 has an amino acid sequence shown at positions 20 to 172 of SEQ ID No. 12, positions 20 to 157 of SEQ ID No. 14, positions 20 to 172 of SEQ ID No. 16, or positions 20 to 209 of SEQ ID No. 18.
Item 5. The fusion protein according to any one of the items 1-4 can self-assemble into a 10-20 nm nanoparticle after being expressed, and is composed of a pentamer of B5 protein and a trimer of Tri.
Item 6. The fusion protein according to any one of the items 1-4 further includes a peptide fragment containing O-glycosylation site of the pilin protein PilE of *Neisseria meningitidis.* Preferably, the peptide fragment is a peptide fragment of PilE starting from the serine at position 45 (S45) of the N terminal to the lysine at position 73 (K73). Preferably, the amino acid sequence of the fusion protein is shown in SEQ ID NO. 15, SEQ ID NO. 17, SEQ ID NO. 19, or SEQ ID NO. 21.
Item 7. A glycoprotein nanoparticle includes a protein-polysaccharide conjugate. The conjugate is formed by coupling a bacterial O-polysaccharide and the fusion protein described in the item 6, and the bacterial polysaccharide is coupled to the fusion protein of the item 6 through the glycosylation site 4573. Preferably, the bacterial O-antigen polysaccharide is selected from *Shigella* O polysaccharide, *Escherichia coli* 01570 polysaccharide, *Shigella dysenteriae* O polysaccharide, *Salmonella* paratyphi A O polysaccharide, and *Vibrio cholerae* O polysaccharide.
Item 8. The glycoprotein nanoparticle according to item 7 has a size of about 25-50 nm.
Item 9. A method for preparing the glycoprotein nanoparticle according to the item 7 or 8 includes the following steps:
   1) constructing a host bacterium lacking O-antigen ligase;
   2) construct a co-expression vector of the fusion protein described in the item 6 and glycosyltransferase PglL, and transferring into the above host bacterium to induce expression; and
   3) purifying the target glycoprotein nanoparticle, preferably, purifying the target glycoprotein nanoparticle by affinity chromatography and molecular sieve.
Item 10. Application of the glycoprotein nanoparticle according to the item 7 or 8 in a product for producing a specific antibody to a bacterial polysaccharide in an animal. Preferably, the product is a vaccine.
Item 11. An immunogenic composition for inducing an immune response to a bacterial polysaccharide, where the immunogenic composition includes an immunologically effective dose of the glycoprotein nanoparticle according to the item 7 or 8. Preferably, the immunogenic composition does not contain an adjuvant.
Item 12. A vaccine for inducing an immune response to a bacterial polysaccharide, where the vaccine includes an immunologically effective dose of the fusion protein according to any one of the items 1-6 and a bacterial polysaccharide, or the glycoprotein nanoparticle according to the item 7 or 8. Preferably, the vaccine does not contain an adjuvant.

### Definition of Terms

In the present invention, unless otherwise specified, the scientific and technical terms used herein have the meanings commonly understood by those skilled in the art. In addition, the microbiology, the operating steps adopted herein associated with microbiology, immunology, biochemistry, nucleic acid chemistry and the like are all routine steps widely used in the corresponding fields. At the same time, in order to better understand the present invention, definitions and explanations of related terms are provided below.

As used herein, the term "O-polysaccharide" refers to the outermost part of lipopolysaccharide, which is composed of dozens of identical oligosaccharide units. O-polysaccharide has antigenic properties, so it is also called O-antigen. O-antigen is a bacterial antigen of the specific polysaccharide of gram-negative bacteria.

As used herein, the term "AB5 toxin B subunit" can be used interchangeably with "B5" or "B5 protein". AB5 toxin produced by bacteria is a proteinaceous exotoxin produced by toxin-producing bacteria and secreted out of bacterial cells. AB5 toxin is composed of one toxic active subunit (A subunit) and five binding subunits (B subunit) with cell binding activity. AB5 toxin B subunit can self-assemble into a pentamer.

As used herein, the term "vector" refers to a nucleic acid delivery vehicle into which a polynucleotide can be inserted. When the vector enables an expression of the protein encoded by the inserted polynucleotide, the vector is called an expression vector. The vector can be introduced into a host cell through transformation, transduction or transfection, so that genetic material elements that are carried can be expressed in the host cell. The vector is well known to those skilled in the art, including but not limited to: plasmids; phagemids; cosmids; artificial chromosomes, such as yeast artificial chromosomes (YAC), bacterial artificial chromosomes (BAC) or P1-derived artificial chromosomes (PAC); bacteriophages such as lambda (λ) phages or M13 phages and animal viruses. Animal viruses that can be used as a vector include, but are not limited to, retroviruses (including lentiviruses), adenoviruses, adeno-associated viruses, herpes viruses (such as herpes simplex virus), poxviruses, baculoviruses, papillomavirus, and papovaviruses (such as SV40). A vector can contain a variety of elements that control expression, including but not limited to promoter sequences, transcription initiation sequences, enhancer sequences, selection elements, and reporter genes. In addition, the vector may also contain a replication origin.

As used herein, the term "host cell" refers to a cell where a vector can be introduced, which includes, but is not limited to, prokaryotic cells such as *Escherichia coli* or *bacillus subtilis,* fungal cells such as yeast cells or *Aspergillus,* insect cells such as *Drosophila* S2 cells or *Spodoptera frugiperda* (Sf9), or animal cells such as fibroblasts, Chinese hamster ovary (CHO) cells, COS cells, NSO cells, HeLa cells, BHK cells, HEK 293 cells or human cells.

The twenty conventional amino acids involved herein are written in a common manner, referring to, for example, Immunology-A Synthesis (2nd Edition, E. S. Golub and D. R. Gren, Eds., Sinauer Associates, Sunderland, Mass. (1991)), which is incorporated herein by reference. In the present invention, the terms "polypeptide" and "protein" have the same meaning and can be used interchangeably. Moreover, in the present invention, amino acids are usually represented by one-letter and three-letter abbreviations that are well known in the art. For example, alanine can be represented as A or Ala.

As used herein, the term "pharmaceutically acceptable carrier and/or excipient" refers to a carrier and/or excipient that is pharmacologically and/or physiologically compatible with a subject and an active ingredient, which is well known in the art (see, for example, Remington's Pharmaceutical Sciences. Edited by Gennaro AR, 19th ed. Pennsylvania: Mack Publishing Company, 1995), and includes, but is not limited to: pH adjusters, surfactants, ionic strength enhancers, agents for maintaining osmotic pressure, agents for delaying absorption, diluents, preservatives, stabilizers, etc. For example, the pH adjusters include, but are not limited to, a phosphate buffer solution. The surfactants include, but are not limited to, cationic, anionic or nonionic surfactants, such as Tween-80. The ionic strength enhancers include, but are not limited to, sodium chloride. The agents for maintaining osmotic pressure include, but are not limited to, sugar, NaCl and analogues thereof. The Agents for delaying absorption include, but are not limited to, monostearate and gelatin. The diluents include, but are not limited to, water, aqueous buffers (such as buffered saline), alcohols and polyols (such as glycerol), etc. The preservatives include, but are not limited to, various antibacterial agents and antifungal agents, such as thimerosal, 2-phenoxyethanol, paraben, chlorobutanol, phenol, sorbic acid and the like. The stabilizers have the meaning commonly understood by those skilled in the art, which can stabilize the desired activity of the active ingredient in a drug, and include, but are not limited to, sodium glutamate, gelatin, SPGA, sugars (such as sorbitol, mannitol, starch, sucrose, lactose, dextran, or glucose), amino acids (such as glutamic acid, glycine), proteins (such as dried whey, albumin or casein) or their degradation products (such as lactalbumin hydrolysate), etc.

As used herein, the term "immunologically effective dose" is an amount that is sufficient to provide a protective immune response to bacterial infections (for example, infections caused by gram-negative pathogenic bacteria) or to induce the body to produce a protective immune response to antigens (for example, bacterial polysaccharide antigen). In some embodiments, the amount should be sufficient to significantly prevent the likelihood or reduce the severity of bacterial infections (for example, infections caused by gram-negative pathogenic bacteria). The immunologically effective dose of the glycoprotein nanoparticle, or the immunogenic composition or vaccine of the present invention may vary with the overall state of a patient's own immune system, the patient's general conditions (such as age, weight, and gender), the method of drug administration, other concurrently used treatments and other factors.

### Beneficial effects

The present inventors discovered for the first time that nanoscale glycoprotein can be obtained by subjecting B5 toxin B subunit and a peptide capable of forming a trimer to a fusion expression and linking a bacterial polysaccharide therein by a glycosylation system. The nanoscale glycoprotein is significantly superior to a single polysaccharide antigen and a polysaccharide conjugate vaccine that only binds to the B5 toxin B subunit carrier protein in terms of the level of antibody induced, bactericidal activity and protective effect. Therefore, the nanoscale glycoprotein of the present invention is particularly suitable for inducing a specific immune response to bacterial O-polysaccharides, and for preventing and/or treating bacterial infections, possessing great clinical value.

### BRIEF DESCRIPTION OF THE DRAWINGS

FIG. 1 shows detection results of a monomer size of CTBTri after purification and a size of a particle formed thereby. A: detection results of Coomassie brilliant blue staining analysis and western blot; B: detection result of dynamic light scattering; C: detection result of transmission electron microscopy.
FIG. 2 shows detection results of a monomer size of StxBTri after expression and purification and a size of a particle formed thereby. A: detection results of Coomassie brilliant blue staining analysis and western blot; B: detection result of dynamic light scattering; C: detection result of transmission electron microscopy.
FIG. 3 shows detection results of a monomer size of LTBTri after expression and purification and a size of a particle formed thereby. A: detection results of Coomassie brilliant blue staining analysis and western blot; B: detection result of dynamic light scattering; C: detection result of transmission electron microscopy.
FIG. 4 shows detection results of a monomer size of SubBTri after expression and purification and a size of a particle formed thereby. A: detection results of Coomassie brilliant blue staining analysis and western blot; B: detection result of dynamic light scattering; C: detection result of transmission electron microscopy.
FIG. 5 shows detection results of expressions of four glycoproteins CTBTri4573-OPS, StxBTri4573-OPS, LTBTri4573-OPS, and SubBTri4573-OPS.
FIG. 6 shows Coomassie brilliant blue staining analysis of four glycoproteins CTBTri4573-OPS, StxBTri4573-OPS, LTBTri4573-OPS, and SubBTri4573-OPS after passing through a molecular sieve.
FIG. 7 shows particle sizes of four glycoproteins CTBTri4573-OPS, StxBTri4573-OPS, LTBTri4573-OPS, and SubBTri4573-OPS detected by dynamic light scattering and transmission electron microscopy analysis. A: dynamic light scattering; B: transmission electron microscopy.
FIG. 8 shows determination results of antibody titer of mice immunized with B5Tri4573-OPS (CTBTri4573-OPS, StxBTri4573-OPS, LTBTri573-OPS, SubBTri4573-OPS). ^{∗}*p*<0.05, ^{∗∗∗}*p*<0.001, ^{∗∗∗∗}*p*<0.0001.
FIG. 9 shows challenge test results of mice immunized with B5Tri4573-OPS (CTBTri4573-OPS, StxBTri4573-OPS, LTBTri573-OPS, SubBTri4573-OPS).
FIG. 10 shows determination results of antibody titer of different IgG subtypes after mice are immunized with CTBTri4573-OPS. ^{∗∗∗∗}*p*<0.0001.
FIG. 11 shows a comparison of antibody titers of different IgG subtypes after mice are immunized with CTBTri4573-OPS with and without an adjuvant. ^{∗}*p*<0.05, ^{∗∗}*p*<0.01.
FIG. 12 shows tests of *in vitro* bactericidal activity of an antiserum induced by CTBTri4573-OPS.
FIG. 13 shows challenge test results of mice immunized with CTBTri4573-OPS.

### Sequence information

**Table 1: Information of sequences involved in the present invention is provided in the table below.**

| SEQ ID NO: | Description | SEQ ID NO: | Description |
|---|---|---|---|
| 1 | Amino acid sequence of CTB | 18 | Amino acid sequence of SubBTri protein |
| 2 | Nucleotide sequence of CTB | 19 | Amino acid sequence of PglL |
| 3 | Amino acid sequence of StxB | 20 | Nucleotide sequence of PglL expression cassette |
| 4 | Nucleotide sequence of StxB | 21 | Amino acid sequence of CTBTri4573 |
| 5 | Amino acid sequence of LTB | 22 | Nucleotide sequence of CTBTri4573 expression cassette |
| 6 | Nucleotide sequence of LTB | 23 | Amino acid sequence of StxBTri4573 |
| 7 | Amino acid sequence of SubB | 24 | Nucleotide sequence of StxBTri4573 expression cassette |
| 8 | Nucleotide sequence of SubB | 25 | Amino acid sequence of LTBTri457 |
| 9 | Amino acid sequence of Tri | 26 | Nucleotide sequence of LTBTri4573 expression cassette |
| 10 | Nucleotide sequence of Tri | 27 | Amino acid sequence of SubBTri4573 |
| 11 | Nucleotide sequence of tacCTBTri expression cassette | 28 | Nucleotide sequence of SubBTri4573 expression cassette |
| 12 | Amino acid sequence of CTBTri protein | 29 | Glycosylation sequence (PilE S45-K73) |
| 13 | Nucleotide sequence of tacStxBTri expression cassette | 30 | Linker |
| 14 | Amino acid sequence of StxBTri protein | 31 | DsbA signal peptide |
| 15 | Nucleotide sequence of tacLTBTri expression cassette | 32 | Primer SacI-UP |
| 16 | Amino acid sequence of LTBTri protein | 33 | Primer XhoI-Down |
| 17 | Nucleotide sequence of tacSubBTri expression cassette | | |

### DETAILED DESCRIPTION OF THE EMBODIMENTS

The experimental methods in the following embodiments, unless otherwise specified, are all conventional methods. The materials, reagents, etc. used in the following embodiments are commercially available unless otherwise specified.

The non-toxic *Shigella flexneri* 301DWP lacking O-antigen ligase gene *waaI* in the following embodiments was obtained by modifying the wild strain of *Shigella flexneri* 2a 301 (S. *flexneri* 2a 301) (Jin Q et al. Nucleic Acids Res. 2002 Oct 15;30(20):4432-41). The public can reach it from the Institute of Bioengineering, Academy of Military Medicine, PLA Academy of Military Sciences. The biological material was only used for repeating the relevant experiments of the present invention, and cannot be used for other purposes.

pET28a(+) in the following embodiments was purchased from Novagen.

HRP-labeled Anti-His antibody in the following embodiments was an Abmart product, with a product number of M20020.

Rabbit-derived Freund's type II serum in the following embodiments was a product of Denka Seiken Co., Ltd., with a number of 210227.

HRP-labeled goat anti-rabbit in the following embodiments was a product of TransGen Biotech Co., LTD, with a number of HS-101-01.

HRP-labeled donkey anti-mouse IgG, goat anti-mouse IgG1, IgG2a, IgG2b, and IgG3 in the following embodiments were purchased from Abcam, with numbers of ab6820, ab97240, ab97245, ab97250, and ab97260, respectively.

Aluminum hydroxide adjuvant Rehydragel LV in the following embodiments was purchased from General Chemical Company, with a number of 203120070602.

Complement in the following embodiments was purchased from Pel-Freez LLC, with a number of 31061-3.

### Embodiment 1. Construction and expression of nanoparticle vector

### 1) Construction of B5Tri fusion protein expression vector

In the present embodiment, a vector for expressing the fusion protein (B5Tri) including B5 protein and Tri sequence was constructed, where the B5 protein included cholera toxin B subunit (CTB), Shiga toxin B subunit (StxB), *Escherichia coli* heat-labile enterotoxin B subunit (LTB), and *Bacillus subtilis* toxin B subunit (SubB), and the Tri sequence was shown in SEQ ID NO. 9. The construction method was as follows:

CTBTri expression vector construction: According to the amino acid sequence of cholera toxin B subunit (X76390.1) published by GeneBack, a signal peptide (first 21 amino acids) was replaced with a DsbA signal peptide (SEQ ID NO. 31), and Tri sequence was fused at the C terminus of CTB to construct CTB fusion protein CTBTri. In the CTBTri expression cassette, the expression of CTBTri was initiated by the tac promoter. The expression cassette was named tacCTBTri, and had a nucleotide sequence shown in SEQ ID No. 11, wherein the nucleotides at positions 103 to 131 were the tac promoter sequence, the nucleotides at positions 178 to 729 were the coding sequence of CTBTri (CTBTri protein sequentially included DsbA signal peptide, CTB, Tri and His tag from N terminal to C terminal). Specifically from the 5' end, the nucleotides at positions 178 to 234 were the signal peptide sequence, the nucleotides at positions 235 to 543 were the CTB coding sequence, and the nucleotides at positions 556 to 693 were the Tri sequence. The amino acid sequence of the CTBTri protein was shown in SEQ ID No. 12, in which from the N terminus, the nucleotides at positions 1 to 19 were the DsbA signal peptide sequence, the nucleotides at positions 20 to 122 were the CTB amino acid sequence, and the nucleotides at positions 123 to 126 were a flexible linker, and the nucleotides at positions 127 to 172 were the Tri sequence.

StxBTri expression vector construction: In the StxBTri expression cassette, the expression of StxBTri was initiated by the tac promoter. The expression cassette was named tacStxBTri, and had a nucleotide sequence shown in SEQ ID No. 13, wherein the nucleotides at positions 103 to 131 were the tac promoter sequence, and the nucleotides at positions 178 to 684 were the coding sequence of StxBTri (StxBTri protein sequentially included DsbA signal peptide, StxB, Tri, and His tag from N terminal to C terminal). Specifically from the 5' end, the nucleotides at positions 178 to 234 were the signal peptide sequence, the nucleotides at positions 235 to 498 were the StxB coding sequence, and the nucleotides at positions 511 to 648 were the Tri sequence. The amino acid sequence of the StxBTri protein was shown in SEQ ID No. 14, in which from the N terminus, the nucleotides at positions 1 to 19 were the DsbA signal peptide sequence, the nucleotides at positions 20 to 107 were the StxB amino acid sequence, the nucleotides at positions 108 to 111 were a flexible linker, and the nucleotides at positions 112 to 157 were the Tri sequence.

LTBTri expression vector construction: In the LTBTri expression cassette, the expression of LTBTri was initiated by the tac promoter. The expression cassette was named tacLTBTri, and had a nucleotide sequence shown in SEQ ID No. 15, wherein the nucleotides at positions 103 to 131 were the tac promoter sequence, and the nucleotides at positions 178 to 729 were the coding sequence of LTBTri (LTBTri protein sequentially included DsbA signal peptide, LTB, Tri, and His tag from N terminal to C terminal). Specifically from the 5' end, the nucleotides at positions 178 to 234 were the signal peptide sequence, the nucleotides at positions 235 to 543 were the LTB coding sequence, and the nucleotides at positions 556 to 693 were the Tri sequence. The amino acid sequence of the LTBTri protein was shown in SEQ ID No. 16, in which from the N terminus, the nucleotides at positions 1 to 19 were the DsbA signal peptide sequence, the nucleotides at positions 20 to 122 were the LTB amino acid sequence, the nucleotides at positions 123 to 126 were a flexible linker, and the nucleotides at positions 127 to 172 were the Tri sequence.

SubBTri expression vector construction: In the SubBTri expression cassette, the expression of SubBTri was initiated by the tac promoter. The expression cassette was named tacSubBTri, and had a nucleotide sequence shown in SEQ ID No. 17, wherein the nucleotides at positions 103 to 131 were the tac promoter sequence, and the nucleotides at positions 178 to 840 were the coding sequence of SubBTri (SubBTri protein sequentially included DsbA signal peptide, SubB, Tri, and His tag from N terminal to C terminal). Specifically from the 5' end, the nucleotides at positions 178 to 234 were the signal peptide sequence, the nucleotides at positions 235 to 654 were the SubB coding sequence, and the nucleotides at positions 667 to 804 were the Tri sequence. The amino acid sequence of the SubBTri protein was shown in SEQ ID No. 18, in which from the N terminus, the nucleotides at positions 1 to 19 were the DsbA signal peptide sequence, the nucleotides at positions 20 to 159 were the SubB amino acid sequence, the nucleotides at positions 160 to 163 were a flexible linker, and the nucleotides at positions 164 to 209 were the Tri sequence.

The DNA molecules shown in SEQ ID No. 11, SEQ ID No. 13, SEQ ID No. 15, and SEQ ID No. 17 were respectively digested with *Xba*I and *XhoI* to obtain gene fragments. pET28a(+) was digested with *Xba*I and *XhoI* to obtain large fragments of the vector. The gene fragments and the large fragments of the vector were ligated to obtain recombinant vectors respectively containing the DNA molecules shown in SEQ ID No. 11, SEQ ID No. 13, SEQ ID No. 15, and SEQ ID No. 17, and the recombinant vectors were named pET28-tacB5Tri (B5 was CTB, StxB, LTB, and SubB, respectively).

### 2) Construction and expression of B5Tri expression strains

The pET28-tacB5Tri vector was transformed into BL21 competence, coated with LB solid medium containing kanamycin at a final concentration of 50 µg/mL, and subjected to a positive clone to obtain BL21/pET28-tacB5Tri. The BL21/pET28-tacB5Tri was selected and inoculated in LB medium containing kanamycin at a final concentration of 50 µg/mL for culture at 37°C and 220 rpm for 10 h, and then transferred to 5 mL of LB medium containing kanamycin at a final concentration of 50 µg/mL for culture at 37°C and 220 rpm until an OD600 reached about 0.6, followed by adding IPTG with a final concentration of 1 mM, reducing a temperature to 30°C, and inducing for 10 h.

1 mL of each bacterial solution induced at 30°C for 10 h was taken and centrifuged to collect bacterial cells. The bacterial cells were suspended with 1X reduction buffer (50 mM pH 6.8 Tris-HCl, 1.6% SDS, 0.02% bromophenol blue, 8% glycerol, 20 mM DTT), and underwent a boiling water bath for 10 min and a 12% sodium dodecyl sulfate polyacrylamide gel electrophoresis (SDS-PAGE). After the electrophoresis, the proteins were transferred to poly (vinylidene fluoride) (PVDF) membrane at a constant voltage of 20 V for 1 h with Bio-Lab semi-dry transfer apparatus. The protein expression bands were detected using mouse-derived HRP-Anti-His tag monoclonal antibodies.

### Embodiment 2. Purification and detection of nanoparticles

The BL21/pET28-tacB5Tri was inoculated in LB medium containing kanamycin at a final concentration of 50 µg/mL for culture at 37°C and 220 rpm for 10 h, and then transferred to 5 L of LB medium for culture at 37°C and 220 rpm until an OD600 reached about 0.6, followed by adding IPTG with a final concentration of 1 mM, reducing a temperature to 30°C, and inducing for 10 h.

Sample treatment: bacterial cells obtained above after an induction at 30°C for 10 h were taken and added with 400 mL of a loading buffer (20 mM pH 7.5 Tris-HCl, 0.2 M NaCl, 10 mM imidazole), ultrasonically broken (ultrasound worked for 3 s and paused for 5 s, with cumulative ultrasonic time of 30 min), and centrifuged at 12000 g to collect a supernatant.

Sample purification: a column bed was first washed with 0.5 M NaOH aqueous solution for at least 3 bed volumes, and then deionized water was used to adjust pH to neutral. Subsequently, 0.5 M NiSO₄ aqueous solution was used to equilibrate at least 3 bed volumes, and then B1 solution (20 mM pH 7.5 Tris-HCl, 0.5 M NaCl, 500 mM imidazole) was used to equilibrate at least one bed volume, and finally A1 solution (20 mM pH 7.5 Tris-HCl, 0.5 M NaCl, 10 mM imidazole) was used to equilibrate at least 3 bed volumes, each of the above was performed at a flow rate of 4 mL/min. A sample was loaded from an A pipeline, washed with A1 solution (20 mM pH 7.5 Tris-HCl, 0.5 M NaCl, 10 mM imidazole) to remove unbound proteins until the ultraviolet absorption (280 nm) was close to 0 mAU, and finally eluted with 100% B1 (20 mM pH 7.5 Tris-HCl, 0.5 M NaCl, 500 mM imidazole). 30 mL of eluate was collected to obtain a purified sample, and then the purified sample passed through a molecular sieve (superdex 200) with a mobile phase of 1×PBS, pH 7.4.

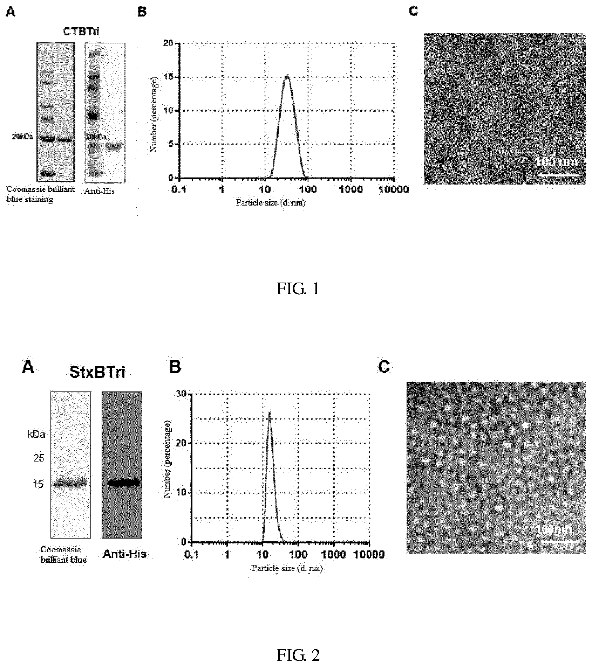

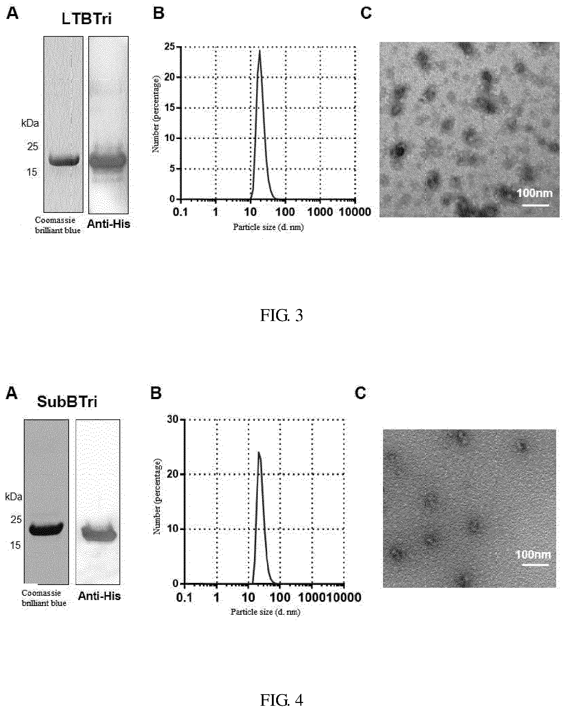

Four fusion proteins (CTBTri, StxBTri, LTBTri, SubBTri) were analyzed by Coomassie brilliant blue staining after passing through molecular sieves (Figure 1A-4A), and protein samples with purity greater than 90% can be obtained. Use His tag antibody to conduct Western blot verification, the detection results of the four fusion proteins are shown in Figure 1A-4A. The sample size was detected by dynamic light scattering and transmission electron microscopy. The dynamic light scattering detection results of the four fusion proteins are shown in Figure 1B-4B, and the transmission electron microscopy detection results of the four fusion proteins are shown in Figure 1C-4C. It can be seen that although the protein monomer is only about 20kDa, it can form protein particles of about 10-20nm in its natural state.

### Embodiment 3. Preparation of nanoscale bacterial polysaccharide conjugate vaccine by biological methods

### 3.1 Construction of B5Tri (B5 was CTB, StxB, LTB, and SubB, rspecitively) glycosylation expression vector

3.1.1 Construction of *Neisseria meningitidis* glycosyltransferase PglL expression vector: the amino acid sequence of the *Neisseria meningitidis* glycosyltransferase PglL (GeneBank: JN200826.1) was shown in SEQ ID No. 19, and its coding sequence included the nucleotides at positions 180 to 1994 of SEQ ID No. 20. The nucleotides at positions 1 to 6 of SEQ ID No. 20 were the *Xba*I recognition site, and the nucleotides at positions 105 to 2240 of SEQ ID No. 20 were the sequence of the PglL expression cassette. In the PglL expression cassette, the expression of PglL was initiated by the tac promoter, and the expression cassette was named tacpglL. Specifically in SEQ ID No. 20, the nucleotides at positions 105 to 133 were the tac promoter sequence, the nucleotides at positions 180 to 1994 were the coding sequence of *Neisseria meningitidis* glycosyltransferase PglL, the nucleotides at positions 2475 to 2480 were the *Sac*I recognition sequence, and the nucleotides at positions 2486 to 2491 were the *Xho*I recognition site.

The DNA molecule shown in SEQ ID No. 20 was digested with *Xba*I and *Xho*I to obtain gene fragments. pET28a(+) was digested with *Xba*I and *Xho*I to obtain large fragments of the vector. The gene fragments and the large fragments of the vector were ligated to obtain a recombinant vector containing the DNA molecule shown in SEQ ID No. 20, and the recombinant vector was named pET28-tacpglL.

3.1.2 Fusion of B5Tri sequence and O-glycosylation site (B5Tri4573): PilE was the pilin of *Neisseria meningitidis.* In *Neisseria meningitidis,* PilE can be O-glycosylated under the catalysis of glycosyltransferase PglL. The glycosylation site was the serine at position 63 of the PilE. In order to obtain the glycosylation sequence, 29 amino acids S45-K73, from the serine (S45) at position 45 of the N terminal of the PilE to the lysine (K73) at position 73, were intercepted and fused to the C terminus of B5Tri; at the same time, in order to avoid steric hindrance effect between B5 and Tri peptide fragment, the B5 and Tri peptide fragment were connected by 4 flexible amino acids.

The amino acid sequence of CTBTri4573 was shown in SEQ ID No. 21, and the gene sequence was shown in positions 178 to 816 of SEQ ID No. 22. In SEQ ID No. 22, the nucleotides at positions 1 to 6 were the *Xba*I recognition site, the nucleotides at positions 103 to 131 were the tac promoter sequence, the nucleotides at positions 178 to 792 were the coding sequence of CTBTri4573, the nucleotides at positions 793 to 798 were the *Xho*I recognition sequence, and the nucleotides at positions 799 to 816 were the His tag.

The amino acid sequence of StxBTri4573 was shown in SEQ ID No. 23, and the gene sequence was shown in positions 178 to 771 of SEQ ID No. 24. In SEQ ID No. 24, the nucleotides at positions 1 to 6 were the *Xba*I recognition site, the nucleotides at positions 103 to 131 were the tac promoter sequence, the nucleotides at positions 178 to 747 were the coding sequence of StxBTri4573, the nucleotides at positions 748 to 753 were the *Xho*I recognition sequence, and the nucleotides at positions 754 to 771 were the His tag.

The amino acid sequence of LTBTri4573 was shown in SEQ ID No. 25, and the gene sequence was shown in positions 178 to 816 of SEQ ID No. 26. In SEQ ID No. 26, the nucleotides at positions 1 to 6 were the *Xba*I recognition site, the nucleotides at positions 103 to 131 were the tac promoter sequence, the nucleotides at positions 178 to 792 were the coding sequence of LTBTri4573, the nucleotides at positions 793 to 798 were the *Xho*I recognition sequence, and the nucleotides at positions 799 to 816 were the His tag.

The amino acid sequence of SubBTri4573 was shown in SEQ ID No. 27, and the gene sequence was shown in positions 178 to 927 of SEQ ID No. 28. In SEQ ID No. 28, the nucleotides at positions 1 to 6 were the *Xba*I recognition site, the nucleotides at positions 103 to 131 were the tac promoter sequence, the nucleotides at positions 178 to 903 were the coding sequence of SubBTri4573, the nucleotides at positions 904 to 909 were the *Xho*I recognition sequence, and the nucleotides at positions 910 to 927 were the His tag.

The DNA molecules shown in SEQ ID No. 22, SEQ ID No. 24, SEQ ID No. 26, and SEQ ID No. 28 were respectively digested with *Xba*I and *Xho*I to obtain gene fragments. pET28a(+) was digested with *Xba*I and *Xho*I to obtain large fragments of the vector. The gene fragments and the large fragments of the vector were ligated to obtain recombinant vectors, and the recombinant vectors were named pET28-tacB5Tri4573 (B5 was CTB, StxB, LTB, and SubB, respectively).

The specific method for constructing B5Tri4573 glycosylation expression vector was as follows:

Primers *Sac*I-UP (SEQ ID NO. 32) and *Xho*I-Down (SEQ ID NO. 33) were respectively used to amplify pET28-tacB5Tri4573 (B5 was CTB, StxB, LTB, and SubB, respectively) to obtain the corresponding amplification products, and the amplification products were digested with *Sac*I and *Xho*I to obtain DNA fragments. pET28-tacpglL was digested with *Sac*I and *Xho*I to obtain large fragments of the vector. The gene fragments were connected to the large fragments of the vector to obtain recombinant plasmids, named pET28- tacpglL-tacB5Tri4573 (B5 was CTB, StxB, LTB, and SubB, respectively), in which the expression cassette (i.e., tacB5Tri4573) composed of the coding gene of B5Tri4573 was located downstream of the pglL expression cassette (tacpglL). The recombinant plasmids were sent for sequencing, and the results were all correct.

### 3.2 Preparation of 301DWP competent cells

The attenuated *Shigella* 301DWP where O-antigen ligase was knocked out was inoculated into LB liquid medium. Since the bacterium lacked O-antigen ligase, free O-antigen can be produced. After overnight culture at 30°C, 1 ml of culture solution was transferred to 100 mL of low-salt LB liquid medium for culture at 30°C until an OD600 reached 0.6. Subsequently, bacterial cells were collected by centrifugation, washed four times with a 10 vol% sterile aqueous glycerol solution, and finally resuspended with 400 uL of the 10 vol% sterile aqueous glycerol solution to obtain 301DWP competent cells for transformation by electroporation, which were sub-packed for use.

### 3.3 Construction of expression strains

The plasmids pET28-tacpglL-tacB5Tri4573 (B5 was CTB, StxB, LTB, and SubB, respectively) were transformed by electroporation into the 301DWP competent cells obtained in 3.2 above, coated with LB solid medium containing kanamycin at a final concentration of 50 µg/mL, and subjected to a positive clone to obtain 301DWP/pET28-tacpglL-tacB5Tri4573 (B5 was CTB, StxB, LTB, and SubB, respectively).

### 3.4 Protein expression and detection

301DWP/pET28-tacpglL-tacB5Tri4573 (B5 was CTB, StxB, LTB, and SubB, respectively) were selected for monoclone, and inoculated in LB medium containing kanamycin at a final concentration of 50 µg/mL for culture at 37°C until an OD600 reached approximately 0.6, followed by adding IPTG having a final concentration of 1 mM, reducing a temperature to 30°C, and inducing for 10 h.

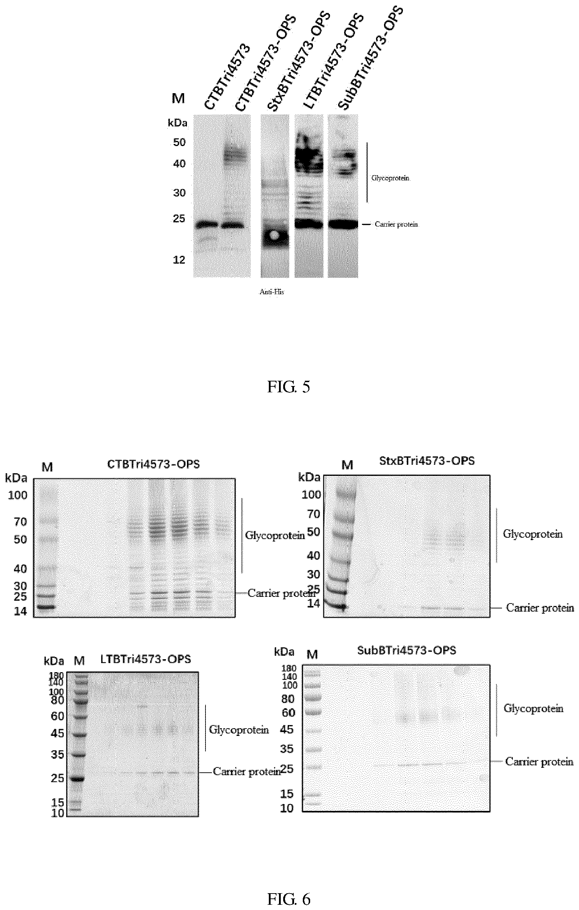

Next day, 1 mL of each bacterial solution induced at 30°C for 10 h above was taken and centrifuged to collect bacterial cells. The bacterial cells were suspended with 1X reduction buffer (50 mM pH 6.8 Tris-HCl, 1.6% SDS, 0.02% bromophenol blue, 8% glycerol, 20 mM DTT), and underwent a boiling water bath for 10 min and a 12% SDS-PAGE. After the electrophoresis, the proteins were transferred to PVDF membrane at a constant voltage of 20 V for 1 h with Bio-Lab semi-dry transfer apparatus, detected by using mouse-derived HRP-Anti-His tag monoclonal antibodies, the results were shown in FIG. 5. The whole bacterial sample was subjected to the SDS-PAGE and western blot (WB) detection in sequence. When PglL and B5Tri4573 were co-expressed, it can be seen from WB bands that upward migration of molecular weight occurred, because the O-antigen polysaccharide of the bacteria itself was transferred to the carrier protein under the catalysis of glycosyltransferase. At the same time, since the bacterial polysaccharide was a tandem repeat structure, a typical glycosylated ladder band was presented, indicating that four carrier proteins (CTBTri4573-OPS, StxBTri4573-OPS, LTBTri4573-OPS, SubBTri4573-OPS) were all glycosylated, and the bacterial polysaccharide was introduced, while the vector without PglL (B5Tri4573) in the control group only showed a carrier protein expression.

### 3.5 Protein purification

The 301DWP/pET28-tacpglL-tacB5Tri4573 (B5 was CTB, StxB, LTB, and SubB, respectively) were inoculated in LB medium containing kanamycin at a final concentration of 50 µg/mL for culture at 37°C and 220 rpm for 10 h, and then transferred to 5 L of LB medium for culture at 37°C and 220 rpm until an OD600 reached about 0.6, followed by adding IPTG with a final concentration of 1 mM, reducing a temperature to 30°C, and inducing for 10 h.

Sample treatment: bacterial cells obtained above after an induction at 30°C for 10 h were taken and added with 400 mL of a loading buffer (20 mM pH 7.5 Tris-HCl, 0.2 M NaCl, 10 mM imidazole), ultrasonically broken (ultrasound worked for 3 s and paused for 5 s, with cumulative ultrasonic time of 30 min), and centrifuged at 12000 g to collect a supernatant.

Sample purification: a column bed was first washed with 0.5 M NaOH aqueous solution for at least 3 bed volumes, and then deionized water was used to adjust pH to neutral. Subsequently, 0.5 M NiSO₄ aqueous solution was used to equilibrate at least 3 bed volumes, and then B1 solution (20 mM pH 7.5 Tris-HCl, 0.5 M NaCl, 500 mM imidazole) was used to equilibrate at least one bed volume, and finally A1 solution (20 mM pH 7.5 Tris-HCl, 0.5 M NaCl, 10 mM imidazole) was used to equilibrate at least 3 bed volumes, each of the above was performed at a flow rate of 4 mL/min. A sample was loaded from an A pipeline, washed with A1 solution (20 mM pH 7.5 Tris-HCl, 0.5 M NaCl, 10 mM imidazole) to remove unbound proteins until the ultraviolet absorption (280 nm) was close to 0 mAU, and finally eluted with 100% B1 (20 mM pH 7.5 Tris-HCl, 0.5 M NaCl, 500 mM imidazole). 30 mL of eluate was collected to obtain a purified sample, and then the purified sample passed through a molecular sieve (superdex 200) with a mobile phase of 1×PBS, pH 7.4.

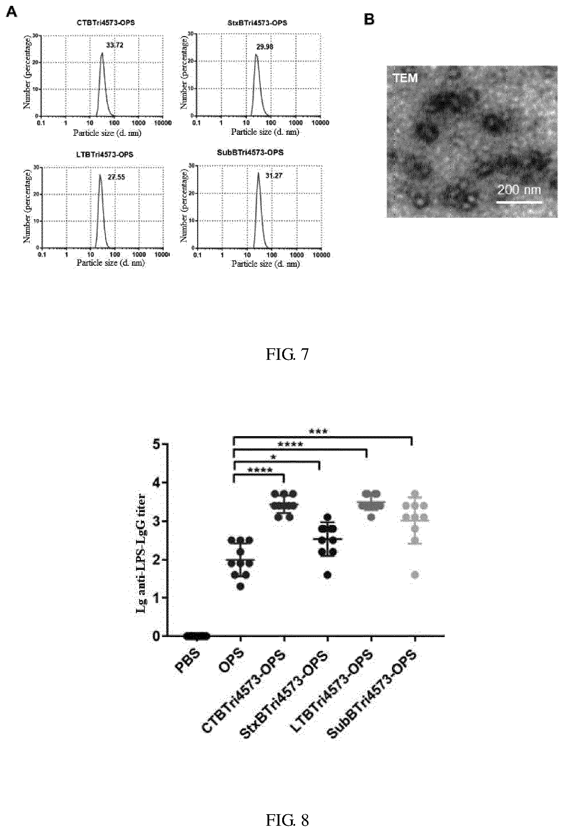

Four glycoproteins (CTBTri4573-OPS, StxBTri4573-OPS, LTBTri4573-OPS, SubBTri4573-OPS) were subjected to a Coomassie brilliant blue staining analysis after passing through a molecular sieve. The results were shown in FIG. 6, and a glycoprotein sample with purity greater than 90% can be obtained. Dynamic light scattering was used to measure the particle size of the sample, and the results were shown in FIG. 7A. All the four glycoproteins can form 25-50 nm glycoprotein particles. The CTBTri4573-OPS was further observed by transmission electron microscope, and the resulting sizes were consistent with the results of dynamic light scattering (FIG. 7B).

### Embodiment 4. Animal immunity evaluation

### 4.1 Evaluation of the effect of B5Tri4573-OPS on immunizing mice

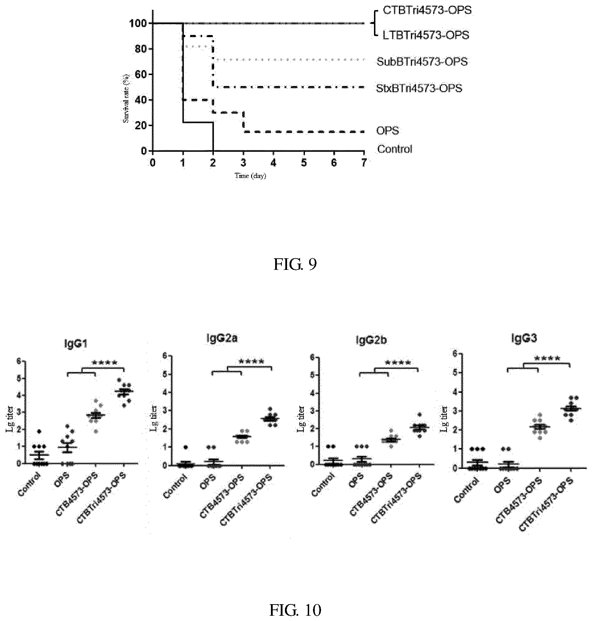

In the present embodiment, the immunological effect of B5Tri4573-OPS was evaluated. B5Tri4573-OPS referred to the glycosylated protein prepared in Embodiment 3 (B5 was CTB, StxB, LTB, and SubB, respectively). Forty 6-week-old female Balb/c mice were taken and randomly divided into 6 groups (PBS group, OPS group (polysaccharide alone), CTBTri4573-OPS group, StxBTri4573-OPS group, LTBTri573-OPS group, and SubBTri4573-OPS group). Each group of mice were injected subcutaneously with 2.5 µg of polysaccharide based on a polysaccharide content; immunized on the 1^{st}, 15^{th}, and 29^{th} day, the tail bloods thereof were collected on the 10^{th} day after the last immunization, and antibody titers of anti*-Shigella flexneri* type 2a O-antigen polysaccharide in the serum of each group of mice were measured by indirect enzyme-linked immunosorbent assay (ELISA). The enzyme-linked plate was coated with the extracted LPS of *anti-Shigella flexneri* type 2a 301 strain, with 10 µg of LPS per well, and other operating steps referred to the "Short Protocols in Molecular Biology". The results were shown in FIG. 8. All the four glycoproteins can induce a generation of specific antibodies in mice, which effectively triggerred the body's immune response, and showed antibody titers induced thereby significantly superior to those in the OPS group administered with polysaccharide alone. To further confirm the above effects, two weeks after the last immunization, the mice were injected intraperitoneally with *Shigella flexneri* 301 wild strains at a dose of 5 times the half lethal dose (4.35×10⁷ colony-forming units (CFU)), namely, 200 µl, followed by observing the death of the mice. The results were shown in FIG. 9. A protective effect was generated in the mice in each group of B5Tri4573-OPS at the dose of 5 times the half lethal dose, and the protection rate of CTBTri573-OPS and LTBTri573-OPS can reach 100%.

### 4.2 Further evaluation of the immunological effect of CTBTri4573-OPS

### 4.2.1 Comparison of the immunological effects of CTBTri4573-OPS and CTB4573-OPS

Forty 6-week-old female Balb/c mice were taken and randomly divided into 4 groups (PBS group (Control), OPS group, CTB4573-OPS, and CTBTri4573-OPS group). Among them, CTB4573-OPS was a glycoprotein prepared with CTB (without Tri sequence) as a substrate protein, and its preparation method referred to Pan C et al., mBio. 2016 Apr 26;7(2):e00443-16. Each group of mice were injected subcutaneously with 2.5 µg of polysaccharide based on a polysaccharide content; immunized on the 1^{st}, 15^{th}, and 29^{th} day, the tail bloods thereof were collected on the 10^{th} day after the last immunization, and antibody titers of *anti-Shigella flexneri* type 2a O-antigen polysaccharide in the serum of each group of mice were measured by indirect ELISA. The enzyme-linked plate was coated with the extracted LPS of *anti-Shigella flexneri* type 2a 301 strain, with 10 µg of LPS per well, and other operating steps referred to the "Short Protocols in Molecular Biology".

The results were shown in FIG. 10. Compared with the OPS group and the CTB4573-OPS group, the total IgG of nanoscale CTBTri4573-OPS was significantly increased, and the IgG1-dominated humoral immunity was mainly triggered. Meanwhile, the levels of IgG2a and IgG3 were also increased significantly, indicating that the cellular immunity was also activated.

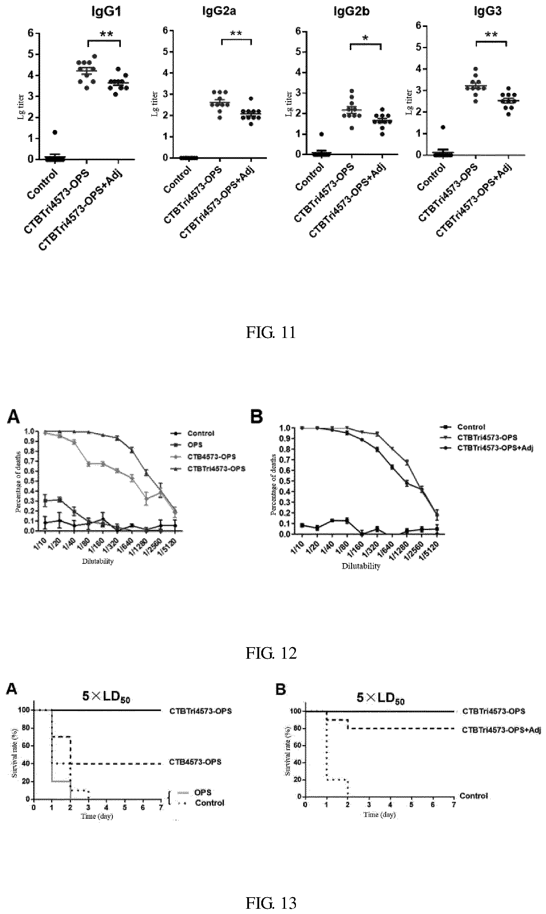

Furthermore, the immunological effects of nanoscale CTBTri4573-OPS with and without an aluminum adjuvant (Adj) were compared. The immunization protocol was the same as that described above, and a dose of the aluminum adjuvant was 10% of the total volume. It was found that the titer of IgG in the nanoscale CTBTri4573-OPS without adjuvant was significantly higher than that with adjuvant group (FIG. 11).

### 4.2.2 Evaluation of bactericidal activity of antibody serum of CTBTri4573-OPS

Regarding whether the antibody in the serum after immunization had bactericidal activity, an *in vitro* bactericidal experiment was further carried out. The *Shigella* 301 wild strains were cultured in LB liquid medium at 37°C in a shaker at 220 rpm until an OD600 reached 2.0, followed by diluting with normal saline to obtain a 100-200 CFU/10 µL dilution. 10 µL of the diluted bacterial solution and 10 µL of immunized mouse serum undergoing a doubling dilution (maintaining in a 56°C water bath for 30 min in advance to inactivate complement components) were mixed and incubated at 37°C for 1 h. After the incubation, 20 µL of complement was added to each tube for well mixing and incubation at 37°C for 1 h. All the resultants were coated on an LB plate, and incubated overnight at 37°C. The total plate count was recorded next day, and the sterilization rate was calculated.

The results were shown in FIG. 12, the bactericidal activity of the antiserum induced by CTBTri4573-OPS was much higher than those induced by polysaccharide alone and CTB4573-OPS, indicating that the protective effect of the nanoscale polysaccharide conjugate vaccine was greatly improved, and a better effect was achieved without adding the adjuvant, thus avoiding the adverse effect caused by the adjuvant.

### 4.2.3 Evaluation of the protection of CTBTri4573-OPS after immunization

To further confirm the above effects, two weeks after the last immunization, the mice were injected intraperitoneally with *Shigella flexneri* 301 wild strains at a dose of 5 times the half lethal dose (4.35×10⁷ CFU), namely, 200 µl, followed by observing the death of the mice. The results were shown in FIG. 13. 100% protection was maintained in the nanoparticle group at the dose of 5 times the half lethal dose, reaching the optimal effect (FIG. 13A). Meanwhile, the protective effect in the group without adding an adjuvant was superior to that with an adjuvant (FIG. 13B).

Although the specific embodiments of the present invention have been described in details, those skilled in the art will understand that various modifications and variations can be made to the details according to all the disclosure published, and these changes are within the protection scope of the present invention. The whole of the present invention is defined by the appended claims and any equivalents thereof.

## Claims

1. A fusion protein, comprising: an AB5 toxin B subunit and a trimerization domain sequence.

2. The fusion protein according to claim 1, wherein the trimerization domain sequence comprises an amino acid sequence shown in SEQ ID No. 9.

3. The fusion protein according to claim 1 or 2, wherein the AB5 toxin B subunit is selected from the group consisting of cholera toxin B subunit (CTB), Shiga toxin B subunit (StxB), *Escherichia coli* heat-labile enterotoxin B subunit (LTB), and *Bacillus subtilis* toxin B subunit (SubB);
preferably, the CTB has an amino acid sequence shown in SEQ ID No. 1; preferably, the StxB has an amino acid sequence shown in SEQ ID No. 3; preferably, the LTB has an amino acid sequence shown in SEQ ID No. 5; preferably, the SubB has an amino acid sequence shown in SEQ ID No. 7.

4. The fusion protein according to any one of claims 1-3, wherein the fusion protein comprises, from N terminus to C terminus, the AB5 toxin B subunit and the trimerization domain sequence.

5. The fusion protein according to claim 4, wherein the fusion protein comprises an amino acid sequence selected from the group consisting of:
(1) an amino acid sequence consisting of amino acid residues at positions 20 to 172 of the sequence shown in SEQ ID NO. 21;
(2) an amino acid sequence consisting of amino acid residues at positions 20 to 157 of the sequence shown in SEQ ID NO. 23;
(3) an amino acid sequence consisting of amino acid residues at positions 20 to 172 of the sequence shown in SEQ ID NO. 25; and
(4) an amino acid sequence consisting of amino acid residues at positions 20 to 209 of the sequence shown in SEQ ID NO. 27.

6. The fusion protein according to any one of claims 1-5, further comprising: a glycosylation sequence containing an O-glycosylation site.

7. The fusion protein according to claim 6, wherein the glycosylation sequence is selected from a peptide fragment containing O-glycosylation site- of the pilin protein PilE of *Neisseria meningitidis;*
preferably, the O-glycosylation site is the serine at position 63 of the PilE.

8. The fusion protein according to claim 7, wherein the glycosylation sequence comprises a peptide fragment consisting of amino acid residues at positions 45 to 73 of the PilE;
preferably, the glycosylation sequence comprises a sequence shown in SEQ ID NO. 29.

9. The fusion protein according to any one of claims 6-8, wherein the fusion protein, from N terminus to C terminus, comprises the AB5 toxin B subunit, the trimerization domain sequence, and the glycosylation sequence.

10. The fusion protein according to claim 9, wherein the fusion protein comprises an amino acid sequence selected from the group consisting of:
(1) an amino acid sequence consisting of amino acid residues at positions 20 to 205 of the sequence shown in SEQ ID NO. 21;
(2) an amino acid sequence consisting of amino acid residues at positions 20 to 190 of the sequence shown in SEQ ID NO. 23;
(3) an amino acid sequence consisting of amino acid residues at positions 20 to 205 of the sequence shown in SEQ ID NO. 25; and
(4) an amino acid sequence consisting of amino acid residues at positions 20 to 242 of the sequence shown in SEQ ID NO. 27.

11. The fusion protein according to any one of claims 1-10, wherein any two adjacent domains are optionally linked by a peptide linker.

12. The fusion protein according to any one of claims 1-11, further comprising a signal peptide and/or a protein tag.

13. An isolated nucleic acid molecule, comprising a nucleotide sequence encoding the fusion protein according to any one of claims 1-12.

14. A vector, comprising the isolated nucleic acid molecule according to claim 13.

15. A host cell, comprising the isolated nucleic acid molecule according to claim 13 or the vector according to claim 14.

16. A method for preparing a glycoprotein nanoparticle, comprising the following steps:
(1) providing a bacterial host cell lacking O-antigen ligase; and
(2) expressing the fusion protein according to any one of claims 6-10 and the glycosyltransferase PglL in the bacterial host cell, thereby producing the target glycoprotein nanoparticle.

17. The method according to claim 16, wherein the bacterial host cell is a pathogenic bacterial cell.

18. The method according to claim 16 or 17, wherein the method further comprises: (3) purifying the target glycoprotein nanoparticle; preferably, purifying the target glycoprotein nanoparticle by affinity chromatography and/or molecular sieve.

19. The method according to any one of claims 16-18, wherein step (2) comprises the following steps:
(2a) introducing a first nucleotide sequence encoding the fusion protein according to any one of claims 6-10 and a second nucleotide sequence encoding PglL into the bacterial host cell, wherein the first nucleotide sequence and the second nucleotide sequence are provided on the same or different expression vectors;
(2b) culturing the bacterial host cell; and
(2c) recovering the target glycoprotein nanoparticle from a cell culture.

20. A glycoprotein nanoparticle, comprising a protein-polysaccharide conjugate, wherein the conjugate is formed by coupling a bacterial O-polysaccharide and the fusion protein according to any one of claims 6-10, and the bacterial O-polysaccharide is coupled to the fusion protein through the O-glycosylation site in the glycosylation sequence in the fusion protein.

21. The glycoprotein nanoparticle according to claim 20, wherein the bacterial O-polysaccharide is selected from pathogenic bacteria O-polysaccharide;
preferably, the bacterial O-polysaccharide is selected from *Shigella* O polysaccharide, *Escherichia coli* 01570 polysaccharide, *Shigella dysenteriae* O polysaccharide, *Salmonella* paratyphi A O polysaccharide, and *Vibrio cholerae* O polysaccharide.

22. The glycoprotein nanoparticle according to claim 20 or 21, **characterized by** one or more of the following characteristics:
(i) in the glycoprotein nanoparticle, the AB5 toxin B subunit of the fusion protein exists in a form of a pentamer, and the trimerization domain sequence of the fusion protein exists in a form of a trimer; and/or
(ii) an average diameter of the glycoprotein nanoparticle is about 25-50 nm.

23. The glycoprotein nanoparticle according to any one of claims 20-22, wherein the glycoprotein nanoparticle is prepared by the method according to any one of claims 16-19.

24. An immunogenic composition or vaccine, comprising: (i) an immunologically effective dose of the glycoprotein nanoparticle according to any one of claims 20-23, or (ii) an immunologically effective dose of the fusion protein according to any one of claims 1-12 or a mixture of a protein nanoparticle formed thereby and bacterial O-polysaccharide;
preferably, the immunogenic composition or vaccine does not comprise an adjuvant;
preferably, the immunogenic composition or vaccine comprises a pharmaceutically acceptable carrier and/or excipient.

25. The application of the glycoprotein nanoparticle according to any one of claims 20-23 or the immunogenic composition or vaccine according to claim 24 in preparing a product for inducing a specific immune response to bacterial O-polysaccharide in a subject and/or for preventing and/or treating bacterial infections in a subject;
preferably, the product is a vaccine;
preferably, the specific immune response comprises an antibody response;
preferably, the subject is a human.

26. The application of the glycoprotein nanoparticle according to any one of claims 20-23 or the immunogenic composition or vaccine according to claim 24 in inducing a specific immune response to bacterial O-polysaccharide in a subject and/or preventing and/or treating bacterial infections in a subject;
preferably, the specific immune response comprises an antibody response;
preferably, the subject is a human.

27. A method for inducing a specific immune response to bacterial O-polysaccharide in a subject or preventing and/or treating a bacterial infection in a subject, comprising administrating an immunologically effective dose of the glycoprotein nanoparticle according to any one of claims 20-23 or the immunogenic composition or vaccine according to claim 24 to a subject in need.


SEQUENCE LISTING

<110>  Academy of Military Medical Sciences

<120>  FUSION PROTEIN CAPABLE OF SELF-ASSEMBLING INTO PROTEIN NANOPARTICLES
AND APPLICATION THEREOF

<130>  IEC190016PCT

<150>  201910352203.X
<151>  2019-04-29

<160>  33

<170>  PatentIn version 3.5

<210>  1
<211>  103
<212>  PRT
<213>  Artificial Sequence

<220>
<223>  Amino acid sequence of CTB

<400>  1

Thr Pro Gln Asn Ile Thr Asp Leu Cys Ala Glu Tyr His Asn Thr Gln
1               5                   10                  15

Ile Tyr Thr Leu Asn Asp Lys Ile Phe Ser Tyr Thr Glu Ser Leu Ala
            20                  25                  30

Gly Lys Arg Glu Met Ala Ile Ile Thr Phe Lys Asn Gly Ala Ile Phe
        35                  40                  45

Gln Val Glu Val Pro Gly Ser Gln His Ile Asp Ser Gln Lys Lys Ala
    50                  55                  60

Ile Glu Arg Met Lys Asp Thr Leu Arg Ile Ala Tyr Leu Thr Glu Ala
65                  70                  75                  80

Lys Val Glu Lys Leu Cys Val Trp Asn Asn Lys Thr Pro His Ala Ile
                85                  90                  95

Ala Ala Ile Ser Met Ala Asn
            100

<210>  2
<211>  309
<212>  DNA
<213>  Artificial Sequence

<220>
<223>  nucleotide sequences of CTB

<400>  2

accccgcaga acatcaccga cctgtgcgcc gagtaccaca acacccaaat ttataccctg 60

aacgacaaaa tttttagcta caccgagagc ctggcaggca agcgcgagat ggccatcatc 120

accttcaaga acggcgccat tttccaggtg gaggtgccgg gcagccagca catcgacagt 180

cagaagaagg ccatcgagcg catgaaggac accctgcgca tcgcctacct gaccgaggcc 240

aaggtggaga agctgtgcgt gtggaacaac aagaccccgc acgccatcgc cgcaatcagc 300

atggccaac 309

<210> 3
<211> 88
<212> PRT
<213> Artificial Sequence

<220>
<223> amino acid sequences of StxB

<400> 3

Lys Lys Thr Leu Leu Ile Ala Ala Ser Leu Ser Phe Phe Ser Ala Ser
1               5                   10                  15

Ala Leu Ala Thr Pro Asp Cys Val Thr Gly Lys Val Glu Tyr Thr Lys
            20                  25                  30

Tyr Asn Asp Asp Asp Thr Phe Thr Val Lys Val Gly Asp Lys Glu Leu
        35                  40                  45

Phe Thr Asn Arg Trp Asn Leu Gln Ser Leu Leu Leu Ser Ala Gln Ile
    50                  55                  60

Thr Gly Met Thr Val Thr Ile Lys Thr Asn Ala Cys His Asn Gly Gly
65                  70                  75                  80

Gly Phe Ser Glu Val Ile Phe Arg
                85

<210> 4
<211> 264
<212> DNA
<213> Artificial Sequence

<220>
<223> nucleotide sequences of StxB

<400> 4
aaaaaaacat tattaatagc tgcatcgctt tcattttttt cagcaagtgc gctggcgacg 60

cctgattgtg taactggaaa ggtggagtat acaaaatata atgatgacga tacctttaca 120

gttaaagtgg gtgataaaga attatttacc aacagatgga atcttcagtc tcttcttctc 180

agtgcgcaaa ttacggggat gactgtaacc attaaaacta atgcctgtca taatggaggg 240

ggattcagcg aagttatttt tcgt                                              264

<210>  5
<211>  103
<212>  PRT
<213>  Artificial Sequence

<220>
<223>  amino acid sequences of LTB

<400>  5

Ala Pro Gln Thr Ile Thr Glu Leu Cys Ser Glu Tyr Arg Asn Thr Gln
1               5                   10                  15

Ile Tyr Thr Ile Asn Asp Lys Ile Leu Ser Tyr Thr Glu Ser Met Ala
            20                  25                  30

Gly Lys Arg Glu Met Val Ile Ile Thr Phe Lys Ser Gly Glu Thr Phe
        35                  40                  45

Gln Val Glu Val Pro Gly Ser Gln His Ile Asp Ser Gln Lys Lys Ala
    50                  55                  60

Ile Glu Arg Met Lys Asp Thr Leu Arg Ile Thr Tyr Leu Thr Glu Thr
65                  70                  75                  80

Lys Ile Asp Lys Leu Cys Val Trp Asn Asn Lys Thr Pro Asn Ser Ile
                85                  90                  95

Ala Ala Ile Ser Met Lys Asn
            100

<210>  6
<211>  309
<212>  DNA
<213>  Artificial Sequence

<220>
<223>  nucleotide sequences of LTB

<400>  6
gcacctcaga ccattaccga actgtgcagt gaatatcgca atacccagat ctataccatt      60

aatgataaga ttctgagcta caccgaaagt atggccggta aacgcgaaat ggttattatt     120

acctttaaga gtggcgaaac ctttcaggtt gaagttccgg gtagccagca tattgatagt     180

cagaaaaaag caattgagcg catgaaagat accctgcgca ttacctatct gaccgaaacc     240

aaaattgata aactgtgcgt ttggaataac aaaaccccga atagcattgc agcaattagt     300

atgaaaaac                                                             309

<210>  7
<211>  140
<212>  PRT
<213>  Artificial Sequence

<220>
<223>  amino acid sequences of SubB

<400>  7

Thr Ile Lys Arg Phe Phe Val Cys Ala Gly Ile Met Gly Cys Leu Ser
1               5                   10                  15

Leu Asn Pro Ala Met Ala Glu Trp Thr Gly Asp Ala Arg Asp Gly Met
            20                  25                  30

Phe Ser Gly Val Val Ile Thr Gln Phe His Thr Gly Gln Ile Asp Asn
        35                  40                  45

Lys Pro Tyr Phe Cys Ile Glu Gly Lys Gln Ser Ala Gly Ser Ser Ile
    50                  55                  60

Ser Ala Cys Ser Met Lys Asn Ser Ser Val Trp Gly Ala Ser Phe Ser
65                  70                  75                  80

Thr Leu Tyr Asn Gln Ala Leu Tyr Phe Tyr Thr Thr Gly Gln Pro Val
                85                  90                  95

Arg Ile Tyr Tyr Glu Pro Gly Val Trp Thr Tyr Pro Pro Phe Val Lys
            100                 105                 110

Ala Leu Thr Ser Asn Ala Leu Val Gly Leu Ser Thr Cys Thr Thr Ser
        115                 120                 125

Thr Glu Cys Phe Gly Pro Asp Arg Lys Lys Asn Ser
    130                 135                 140

<210>  8
<211>  420
<212>  DNA
<213>  Artificial Sequence

<220>
<223>  nucleotide sequences of SubB

<400>  8
accattaagc gctttttcgt gtgtgcaggc attatgggtt gtctgagcct gaatccggca      60

atggccgaat ggaccggcga tgcacgtgat ggcatgttta gcggtgttgt gattacccag     120

tttcataccg gtcagattga taataagccg tatttttgta tcgagggcaa acagagtgcc     180

ggtagtagca ttagtgcatg tagcatgaaa aatagcagtg tgtggggcgc cagctttagt     240

accctgtata atcaggcact gtatttttat accaccggtc agccggtgcg catctattat      300

gaaccgggcg tgtggaccta tccgccgttt gtgaaagcac tgaccagtaa tgccctggtt      360

ggcctgagca cctgcaccac cagcaccgaa tgttttggtc cggatcgtaa aaagaatagt      420

<210>  9
<211>  46
<212>  PRT
<213>  Artificial Sequence

<220>
<223>  amino acid sequences of Tri

<400>  9

Arg Leu Leu Leu Arg Leu Glu Glu Leu Glu Arg Arg Leu Glu Glu Leu
1               5                   10                  15

Ala Lys Phe Val Ala Ala Trp Thr Leu Lys Ala Ala Ala Val Asp Leu
            20                  25                  30

Glu Leu Ala Ala Leu Arg Arg Arg Leu Glu Glu Leu Ala Arg
        35                  40                  45

<210>  10
<211>  138
<212>  PRT
<213>  Artificial Sequence

<220>
<223>  nucleotide sequences of Tri

<400>  10

Cys Gly Cys Cys Thr Gly Cys Thr Gly Cys Thr Gly Cys Gly Thr Cys
1               5                   10                  15

Thr Gly Gly Ala Ala Gly Ala Ala Cys Thr Gly Gly Ala Ala Cys Gly
            20                  25                  30

Thr Cys Gly Cys Cys Thr Gly Gly Ala Ala Gly Ala Gly Cys Thr Gly
        35                  40                  45

Gly Cys Ala Ala Ala Gly Thr Thr Cys Gly Thr Gly Gly Cys Ala Gly
    50                  55                  60

Cys Ala Thr Gly Gly Ala Cys Cys Cys Thr Gly Ala Ala Ala Gly Cys
65                  70                  75                  80

Gly Gly Cys Thr Gly Cys Gly Gly Thr Thr Gly Ala Cys Cys Thr Gly
                85                  90                  95

Gly Ala Ala Cys Thr Gly Gly Cys Thr Gly Cys Thr Cys Thr Gly Cys
            100                 105                 110

Gly Thr Cys Gly Thr Cys Gly Thr Cys Thr Gly Gly Ala Gly Gly Ala
        115                 120                 125

Gly Cys Thr Gly Gly Cys Thr Cys Gly Thr
    130                 135

<210>  11
<211>  729
<212>  DNA
<213>  Artificial Sequence

<220>
<223>  nucleotide sequences of tacCTBTri expression cassette

<400>  11
tctagatgca taattcgtgt cgctcaaggc gcactcccgt tctggataat gttttttgcg      60

ccgacatcat aacggttctg gcaaatattc tgaaatgagc tgttgacaat taatcatcgg     120

ctcgtataat gtgtggaatt gtgagcggat aacaatttca cacaggaaac agaattcatg     180

aagaaaattt ggctggcctt agccggcctg gttctggcat tcagcgccag cgcaaccccg     240

cagaacatca ccgacctgtg cgccgagtac cacaacaccc aaatttatac cctgaacgac     300

aaaattttta gctacaccga gagcctggca ggcaagcgcg agatggccat catcaccttc     360

aagaacggcg ccattttcca ggtggaggtg ccgggcagcc agcacatcga cagtcagaag     420

aaggccatcg agcgcatgaa ggacaccctg cgcatcgcct acctgaccga ggccaaggtg     480

gagaagctgt gcgtgtggaa caacaagacc ccgcacgcca tcgccgcaat cagcatggcc     540

aacggcggat ccggccgcct gctgctgcgt ctggaagaac tggaacgtcg cctggaagag     600

ctggcaaagt tcgtggcagc atggaccctg aaagcggctg cggttgacct ggaactggct     660

gctctgcgtc gtcgtctgga ggagctggct cgtggcggtt ctggtctcga gcaccaccac     720

caccaccac                                                             729

<210>  12
<211>  184
<212>  PRT
<213>  Artificial Sequence

<220>
<223>  amino acid sequences of  CTBTri protein

<400>  12

Met Lys Lys Ile Trp Leu Ala Leu Ala Gly Leu Val Leu Ala Phe Ser
1               5                   10                  15

Ala Ser Ala Thr Pro Gln Asn Ile Thr Asp Leu Cys Ala Glu Tyr His
            20                  25                  30

Asn Thr Gln Ile Tyr Thr Leu Asn Asp Lys Ile Phe Ser Tyr Thr Glu
        35                  40                  45

Ser Leu Ala Gly Lys Arg Glu Met Ala Ile Ile Thr Phe Lys Asn Gly
    50                  55                  60

Ala Ile Phe Gln Val Glu Val Pro Gly Ser Gln His Ile Asp Ser Gln
65                  70                  75                  80

Lys Lys Ala Ile Glu Arg Met Lys Asp Thr Leu Arg Ile Ala Tyr Leu
                85                  90                  95

Thr Glu Ala Lys Val Glu Lys Leu Cys Val Trp Asn Asn Lys Thr Pro
            100                 105                 110

His Ala Ile Ala Ala Ile Ser Met Ala Asn Gly Gly Ser Gly Arg Leu
        115                 120                 125

Leu Leu Arg Leu Glu Glu Leu Glu Arg Arg Leu Glu Glu Leu Ala Lys
    130                 135                 140

Phe Val Ala Ala Trp Thr Leu Lys Ala Ala Ala Val Asp Leu Glu Leu
145                 150                 155                 160

Ala Ala Leu Arg Arg Arg Leu Glu Glu Leu Ala Arg Gly Gly Ser Gly
                165                 170                 175

Leu Glu His His His His His His
            180

<210>  13
<211>  684
<212>  DNA
<213>  Artificial Sequence

<220>
<223>  nucleotide sequences of tacStxBTri expression cassette

<400>  13
tctagatgca taattcgtgt cgctcaaggc gcactcccgt tctggataat gttttttgcg      60

ccgacatcat aacggttctg gcaaatattc tgaaatgagc tgttgacaat taatcatcgg     120

ctcgtataat gtgtggaatt gtgagcggat aacaatttca cacaggaaac agaattcatg     180

aagaaaattt ggctggcctt agccggcctg gttctggcat tcagcgccag cgcaaaaaaa     240

acattattaa tagctgcatc gctttcattt ttttcagcaa gtgcgctggc gacgcctgat     300

tgtgtaactg gaaaggtgga gtatacaaaa tataatgatg acgatacctt tacagttaaa 360

gtgggtgata aagaattatt taccaacaga tggaatcttc agtctcttct tctcagtgcg 420

caaattacgg ggatgactgt aaccattaaa actaatgcct gtcataatgg agggggattc 480

agcgaagtta tttttcgtgg cggatccggc cgcctgctgc tgcgtctgga agaactggaa 540

cgtcgcctgg aagagctggc aaagttcgtg gcagcatgga ccctgaaagc ggctgcggtt 600

gacctggaac tggctgctct gcgtcgtcgt ctggaggagc tggctcgtgg cggttctggt 660

ctcgagcacc accaccacca ccac 684

<210> 14
<211> 169
<212> PRT
<213> Artificial Sequence

<220>
<223> amino acid sequences of StxBTri protein

<400> 14

Met Lys Lys Ile Trp Leu Ala Leu Ala Gly Leu Val Leu Ala Phe Ser
1               5                   10                  15

Ala Ser Ala Lys Lys Thr Leu Leu Ile Ala Ala Ser Leu Ser Phe Phe
            20                  25                  30

Ser Ala Ser Ala Leu Ala Thr Pro Asp Cys Val Thr Gly Lys Val Glu
        35                  40                  45

Tyr Thr Lys Tyr Asn Asp Asp Asp Thr Phe Thr Val Lys Val Gly Asp
    50                  55                  60

Lys Glu Leu Phe Thr Asn Arg Trp Asn Leu Gln Ser Leu Leu Leu Ser
65                  70                  75                  80

Ala Gln Ile Thr Gly Met Thr Val Thr Ile Lys Thr Asn Ala Cys His
                85                  90                  95

Asn Gly Gly Gly Phe Ser Glu Val Ile Phe Arg Gly Gly Ser Gly Arg
            100                 105                 110

Leu Leu Leu Arg Leu Glu Glu Leu Glu Arg Arg Leu Glu Glu Leu Ala
        115                 120                 125

Lys Phe Val Ala Ala Trp Thr Leu Lys Ala Ala Ala Val Asp Leu Glu
    130                 135                 140

Leu Ala Ala Leu Arg Arg Arg Leu Glu Glu Leu Ala Arg Gly Gly Ser
145                 150                 155                 160

Gly Leu Glu His His His His His His
                165

<210>  15
<211>  729
<212>  DNA
<213>  Artificial Sequence

<220>
<223> nucleotide sequences of  tacLTBTri expression cassette

<400>  15
tctagatgca taattcgtgt cgctcaaggc gcactcccgt tctggataat gttttttgcg      60

ccgacatcat aacggttctg gcaaatattc tgaaatgagc tgttgacaat taatcatcgg      120

ctcgtataat gtgtggaatt gtgagcggat aacaatttca cacaggaaac agaattcatg      180

aagaaaattt ggctggcctt agccggcctg gttctggcat tcagcgccag cgcagcacct      240

cagaccatta ccgaactgtg cagtgaatat cgcaataccc agatctatac cattaatgat      300

aagattctga gctacaccga aagtatggcc ggtaaacgcg aaatggttat tattaccttt      360

aagagtggcg aaacctttca ggttgaagtt ccgggtagcc agcatattga tagtcagaaa      420

aaagcaattg agcgcatgaa agataccctg cgcattacct atctgaccga aaccaaaatt      480

gataaactgt gcgtttggaa taacaaaacc ccgaatagca ttgcagcaat tagtatgaaa      540

aacggcggat ccggccgcct gctgctgcgt ctggaagaac tggaacgtcg cctggaagag      600

ctggcaaagt tcgtggcagc atggaccctg aaagcggctg cggttgacct ggaactggct      660

gctctgcgtc gtcgtctgga ggagctggct cgtggcggtt ctggtctcga gcaccaccac      720

caccaccac                                                              729

<210>  16
<211>  184
<212>  PRT
<213>  Artificial Sequence

<220>
<223>  amino acid sequences of LTBTri protein

<400>  16

Met Lys Lys Ile Trp Leu Ala Leu Ala Gly Leu Val Leu Ala Phe Ser
1               5                   10                  15

Ala Ser Ala Ala Pro Gln Thr Ile Thr Glu Leu Cys Ser Glu Tyr Arg
            20                  25                  30

Asn Thr Gln Ile Tyr Thr Ile Asn Asp Lys Ile Leu Ser Tyr Thr Glu
        35                  40                  45

Ser Met Ala Gly Lys Arg Glu Met Val Ile Ile Thr Phe Lys Ser Gly
    50                  55                  60

Glu Thr Phe Gln Val Glu Val Pro Gly Ser Gln His Ile Asp Ser Gln
65                  70                  75                  80

Lys Lys Ala Ile Glu Arg Met Lys Asp Thr Leu Arg Ile Thr Tyr Leu
                85                  90                  95

Thr Glu Thr Lys Ile Asp Lys Leu Cys Val Trp Asn Asn Lys Thr Pro
            100                 105                 110

Asn Ser Ile Ala Ala Ile Ser Met Lys Asn Gly Gly Ser Gly Arg Leu
        115                 120                 125

Leu Leu Arg Leu Glu Glu Leu Glu Arg Arg Leu Glu Glu Leu Ala Lys
    130                 135                 140

Phe Val Ala Ala Trp Thr Leu Lys Ala Ala Ala Val Asp Leu Glu Leu
145                 150                 155                 160

Ala Ala Leu Arg Arg Arg Leu Glu Glu Leu Ala Arg Gly Gly Ser Gly
                165                 170                 175

Leu Glu His His His His His His
            180

<210>  17
<211>  840
<212>  DNA
<213>  Artificial Sequence

<220>
<223>  nucleotide sequences of tacSubBTri expression cassette

<400>  17
tctagatgca taattcgtgt cgctcaaggc gcactcccgt tctggataat gttttttgcg      60

ccgacatcat aacggttctg gcaaatattc tgaaatgagc tgttgacaat taatcatcgg     120

ctcgtataat gtgtggaatt gtgagcggat aacaatttca cacaggaaac agaattcatg     180

aagaaaattt ggctggcctt agccggcctg gttctggcat tcagcgccag cgcaaccatt     240

aagcgctttt tcgtgtgtgc aggcattatg ggttgtctga gcctgaatcc ggcaatggcc     300

gaatggaccg gcgatgcacg tgatggcatg tttagcggtg ttgtgattac ccagtttcat     360

accggtcaga ttgataataa gccgtatttt tgtatcgagg gcaaacagag tgccggtagt     420

agcattagtg catgtagcat gaaaaatagc agtgtgtggg gcgccagctt tagtaccctg     480

tataatcagg cactgtattt ttataccacc ggtcagccgg tgcgcatcta ttatgaaccg     540

ggcgtgtgga cctatccgcc gtttgtgaaa gcactgacca gtaatgccct ggttggcctg 600

agcacctgca ccaccagcac cgaatgtttt ggtccggatc gtaaaaagaa tagtggcgga 660

tccggccgcc tgctgctgcg tctggaagaa ctggaacgtc gcctggaaga gctggcaaag 720

ttcgtggcag catggaccct gaaagcggct gcggttgacc tggaactggc tgctctgcgt 780

cgtcgtctgg aggagctggc tcgtggcggt tctggtctcg agcaccacca ccaccaccac 840

<210> 18
<211> 221
<212> PRT
<213> Artificial Sequence

<220>
<223> amino acid sequences of SubBTri protein

<400> 18

Met Lys Lys Ile Trp Leu Ala Leu Ala Gly Leu Val Leu Ala Phe Ser
1               5                   10                  15

Ala Ser Ala Thr Ile Lys Arg Phe Phe Val Cys Ala Gly Ile Met Gly
            20                  25                  30

Cys Leu Ser Leu Asn Pro Ala Met Ala Glu Trp Thr Gly Asp Ala Arg
        35                  40                  45

Asp Gly Met Phe Ser Gly Val Val Ile Thr Gln Phe His Thr Gly Gln
    50                  55                  60

Ile Asp Asn Lys Pro Tyr Phe Cys Ile Glu Gly Lys Gln Ser Ala Gly
65                  70                  75                  80

Ser Ser Ile Ser Ala Cys Ser Met Lys Asn Ser Ser Val Trp Gly Ala
                85                  90                  95

Ser Phe Ser Thr Leu Tyr Asn Gln Ala Leu Tyr Phe Tyr Thr Thr Gly
            100                 105                 110

Gln Pro Val Arg Ile Tyr Tyr Glu Pro Gly Val Trp Thr Tyr Pro Pro
        115                 120                 125

Phe Val Lys Ala Leu Thr Ser Asn Ala Leu Val Gly Leu Ser Thr Cys
    130                 135                 140

Thr Thr Ser Thr Glu Cys Phe Gly Pro Asp Arg Lys Lys Asn Ser Gly
145                 150                 155                 160

Gly Ser Gly Arg Leu Leu Leu Arg Leu Glu Glu Leu Glu Arg Arg Leu

165                 170                 175

Glu Glu Leu Ala Lys Phe Val Ala Ala Trp Thr Leu Lys Ala Ala Ala
            180                 185                 190

Val Asp Leu Glu Leu Ala Ala Leu Arg Arg Arg Leu Glu Glu Leu Ala
        195                 200                 205

Arg Gly Gly Ser Gly Leu Glu His His His His His His
    210                 215                 220

<210>  19
<211>  604
<212>  PRT
<213>  Artificial Sequence

<220>
<223> amino acid sequences of  PglL

<400>  19

Met Pro Ala Glu Thr Thr Val Ser Gly Ala His Pro Ala Ala Lys Leu
1               5                   10                  15

Pro Ile Tyr Ile Leu Pro Cys Phe Leu Trp Ile Gly Ile Val Pro Phe
            20                  25                  30

Thr Phe Ala Leu Lys Leu Lys Pro Ser Pro Asp Phe Tyr His Asp Ala
        35                  40                  45

Ala Ala Ala Ala Gly Leu Ile Val Leu Leu Phe Leu Thr Ala Gly Lys
    50                  55                  60

Lys Leu Phe Asp Val Lys Ile Pro Ala Ile Ser Phe Leu Leu Phe Ala
65                  70                  75                  80

Met Ala Ala Phe Trp Tyr Leu Gln Ala Arg Leu Met Asn Leu Ile Tyr
                85                  90                  95

Pro Gly Met Asn Asp Ile Val Ser Trp Ile Phe Ile Leu Leu Ala Val
            100                 105                 110

Ser Ala Trp Ala Cys Arg Ser Leu Val Ala His Phe Gly Gln Glu Arg
        115                 120                 125

Ile Val Thr Leu Phe Ala Trp Ser Leu Leu Ile Gly Ser Leu Leu Gln
    130                 135                 140

Ser Cys Ile Val Val Ile Gln Phe Ala Gly Trp Glu Asp Thr Pro Leu
145                 150                 155                 160

Phe Gln Asn Ile Ile Val Tyr Ser Gly Gln Gly Val Ile Gly His Ile
                165                 170                 175

Gly Gln Arg Asn Asn Leu Gly His Tyr Leu Met Trp Gly Ile Leu Ala
            180                 185                 190

Ala Ala Tyr Leu Asn Gly Gln Arg Lys Ile Pro Ala Ala Leu Gly Val
        195                 200                 205

Ile Cys Leu Ile Met Gln Thr Ala Val Leu Gly Leu Val Asn Ser Arg
    210                 215                 220

Thr Ile Leu Thr Tyr Ile Ala Ala Ile Ala Leu Ile Leu Pro Phe Trp
225                 230                 235                 240

Tyr Phe Arg Ser Asp Lys Ser Asn Arg Arg Thr Met Leu Gly Ile Ala
                245                 250                 255

Ala Ala Val Phe Leu Thr Ala Leu Phe Gln Phe Ser Met Asn Thr Ile
            260                 265                 270

Leu Glu Thr Phe Thr Gly Ile Arg Tyr Glu Thr Ala Val Glu Arg Val
        275                 280                 285

Ala Asn Gly Gly Phe Thr Asp Leu Pro Arg Gln Ile Glu Trp Asn Lys
    290                 295                 300

Ala Leu Ala Ala Phe Gln Ser Ala Pro Ile Phe Gly His Gly Trp Asn
305                 310                 315                 320

Ser Phe Ala Gln Gln Thr Phe Leu Ile Asn Ala Glu Gln His Asn Ile
                325                 330                 335

Tyr Asp Asn Leu Leu Ser Asn Leu Phe Thr His Ser His Asn Ile Val
            340                 345                 350

Leu Gln Leu Leu Ala Glu Met Gly Ile Ser Gly Thr Leu Leu Val Ala
        355                 360                 365

Ala Thr Leu Leu Thr Gly Ile Ala Gly Leu Leu Lys Arg Pro Leu Thr
    370                 375                 380

Pro Ala Ser Leu Phe Leu Ile Cys Thr Leu Ala Val Ser Met Cys His
385                 390                 395                 400

Ser Met Leu Glu Tyr Pro Leu Trp Tyr Val Tyr Phe Leu Ile Pro Phe

405                    410                    415

Gly Leu Met Leu Phe Leu Ser Pro Ala Glu Ala Ser Asp Gly Ile Ala
            420                 425                 430

Phe Lys Lys Ala Ala Asn Leu Gly Ile Leu Thr Ala Ser Ala Ala Ile
        435                 440                 445

Phe Ala Gly Leu Leu His Leu Asp Trp Thr Tyr Thr Arg Leu Val Asn
    450                 455                 460

Ala Phe Ser Pro Ala Thr Asp Asp Ser Ala Lys Thr Leu Asn Arg Lys
465                 470                 475                 480

Ile Asn Glu Leu Arg Tyr Ile Ser Ala Asn Ser Pro Met Leu Ser Phe
                485                 490                 495

Tyr Ala Asp Phe Ser Leu Val Asn Phe Ala Leu Pro Glu Tyr Pro Glu
            500                 505                 510

Thr Gln Thr Trp Ala Glu Glu Ala Thr Leu Lys Ser Leu Lys Tyr Arg
        515                 520                 525

Pro His Ser Ala Thr Tyr Arg Ile Ala Leu Tyr Leu Met Arg Gln Gly
    530                 535                 540

Lys Val Ala Glu Ala Lys Gln Trp Met Arg Ala Thr Gln Ser Tyr Tyr
545                 550                 555                 560

Pro Tyr Leu Met Pro Arg Tyr Ala Asp Glu Ile Arg Lys Leu Pro Val
                565                 570                 575

Trp Ala Pro Leu Leu Pro Glu Leu Leu Lys Asp Cys Lys Ala Phe Ala
            580                 585                 590

Ala Ala Pro Gly His Pro Glu Ala Lys Pro Cys Lys
        595                 600

<210>  20
<211>  2491
<212>  DNA
<213>  Artificial Sequence

<220>
<223>  nucleotide sequences of PglL expression cassette

<400>  20
tctagaactg cataattcgt gtcgctcaag gcgcactccc gttctggata atgttttttg       60

cgccgacatc ataacggttc tggcaaatat tctgaaatga gctgttgaca attaatcatc      120

ggctcgtata atgtgtggaa ttgtgagcgg ataacaattt cacacaggaa acagaattca 180

tgcccgctga aacgaccgta tccggcgcgc accccgccgc caaactgccg atttacatcc 240

tgccctgctt cctttggata ggcatcgtcc cctttacctt cgcgctcaaa ctgaaaccgt 300

cgcccgactt ttaccacgat gccgccgccg cagccggcct gattgtcctg ttgttcctca 360

cggcaggaaa aaaactgttt gatgtcaaaa tccccgccat cagcttcctt ctgtttgcaa 420

tggcggcgtt ttggtatctt caggcacgcc tgatgaacct gatttacccc ggtatgaacg 480

acatcgtctc ttggattttc atcttgctcg ccgtcagcgc gtgggcctgc cggagcttgg 540

tcgcacactt cggacaagaa cgcatcgtga ccctgtttgc ctggtcgctg cttatcggct 600

ccctgcttca atcctgcatc gtcgtcatcc agtttgccgg ctgggaagac acccctctgt 660

ttcaaaacat catcgtttac agcgggcaag gcgtaatcgg acacatcggg cagcgcaaca 720

acctcggaca ctacctcatg tggggcatac tcgccgccgc ctacctcaac ggacaacgaa 780

aaatccccgc cgccctcggc gtaatctgcc tgattatgca gaccgccgtt ttaggtttgg 840

tcaactcgcg caccatcttg acctacatag ccgccatcgc cctcatcctt cccttctggt 900

atttccgttc ggacaaatcc aacaggcgga cgatgctcgg catagccgca gccgtattcc 960

ttaccgcgct gttccaattt tccatgaaca ccattctgga aacctttact ggcatccgct 1020

acgaaactgc cgtcgaacgc gtcgccaacg gcggtttcac agacttgccg cgccaaatcg 1080

aatggaataa agcccttgcc gccttccagt ccgccccgat attcgggcac ggctggaaca 1140

gttttgccca acaaaccttc ctcatcaatg ccgaacagca caacatatac gacaacctcc 1200

tcagcaactt gttcacccat tcccacaaca tcgtcctcca actccttgca gagatgggaa 1260

tcagcggcac gcttctggtt gccgcaaccc tgctgacggg cattgccggg ctgcttaaac 1320

gccccctgac ccccgcatcg cttttcctaa tctgcacgct tgccgtcagt atgtgccaca 1380

gtatgctcga atatcctttg tggtatgtct atttcctcat ccctttcgga ctgatgctct 1440

tcctgtcccc cgcagaggct tcagacggca tcgccttcaa aaaagccgcc aatctcggca 1500

tactgaccgc ctccgccgcc atattcgcag gattgctgca cttggactgg acatacaccc 1560

ggctggttaa cgccttttcc cccgccactg acgacagtgc caaaaccctc aaccggaaaa 1620

tcaacgagtt gcgctatatt tccgcaaaca gtccgatgct gtccttttat gccgacttct 1680

ccctcgtaaa cttcgccctg ccggaatacc ccgaaaccca gacttgggcg gaagaagcaa 1740

ccctcaaatc actaaaatac cgcccccact ccgccaccta ccgcatcgcc ctctacctga 1800

tgcggcaagg caaagttgca gaagcaaaac aatggatgcg ggcgacacag tcctattacc 1860

cctacctgat gccccgatac gccgacgaaa tccgcaaact gcccgtatgg gcgccgctgc 1920

tacccgaact gctcaaagac tgcaaagcct tcgccgccgc gcccggtcat ccggaagcaa 1980

aaccctgcaa atgaaagctt ggctgttttg gcggatgaga gaagattttc agcctgatac 2040

agattaaatc agaacgcaga agcggtctga taaaacagaa tttgcctggc ggcagtagcg 2100

cggtggtccc acctgacccc atgccgaact cagaagtgaa acgccgtagc gccgatggta 2160

gtgtggggtc tccccatgcg agagtaggga actgccaggc atcaaataaa acgaaaggct 2220

cagtcgaaag actgggcctt tcgttttatc tgttgtttgt cggtgaacgc tctcctgagt 2280

aggacaaatc cgccgggagc ggatttgaac gttgcgaagc aacggcccgg agggtggcgg 2340

gcaggacgcc cgccataaac tgccaggcat caaattaagc agaaggccat cctgacggat 2400

ggcctttttg cgtttctaca aactcttttg tttatttttc taaatacatt caaatatgta 2460

tccgctcatg agacgagctc ggccgctcga g 2491

<210> 21
<211> 213
<212> PRT
<213> Artificial Sequence

<220>
<223> amino acid sequences of CTBTri4573

<400> 21

Met Lys Lys Ile Trp Leu Ala Leu Ala Gly Leu Val Leu Ala Phe Ser
1               5                   10                  15

Ala Ser Ala Thr Pro Gln Asn Ile Thr Asp Leu Cys Ala Glu Tyr His
            20                  25                  30

Asn Thr Gln Ile Tyr Thr Leu Asn Asp Lys Ile Phe Ser Tyr Thr Glu
        35                  40                  45

Ser Leu Ala Gly Lys Arg Glu Met Ala Ile Ile Thr Phe Lys Asn Gly
    50                  55                  60

Ala Ile Phe Gln Val Glu Val Pro Gly Ser Gln His Ile Asp Ser Gln
65                  70                  75                  80

Lys Lys Ala Ile Glu Arg Met Lys Asp Thr Leu Arg Ile Ala Tyr Leu
                85                  90                  95

Thr Glu Ala Lys Val Glu Lys Leu Cys Val Trp Asn Asn Lys Thr Pro
            100                 105                 110

His Ala Ile Ala Ala Ile Ser Met Ala Asn Gly Gly Ser Gly Arg Leu
        115                 120                 125

Leu Leu Arg Leu Glu Glu Leu Glu Arg Arg Leu Glu Glu Leu Ala Lys
    130                 135                 140

Phe Val Ala Ala Trp Thr Leu Lys Ala Ala Ala Val Asp Leu Glu Leu
145                 150                 155                 160

Ala Ala Leu Arg Arg Arg Leu Glu Glu Leu Ala Arg Gly Gly Ser Gly
                165                 170                 175

Ser Ala Val Thr Glu Tyr Tyr Leu Asn His Gly Glu Trp Pro Gly Asn
            180                 185                 190

Asn Thr Ser Ala Gly Val Ala Thr Ser Ser Glu Ile Lys Leu Glu His
        195                 200                 205

His His His His His
    210

<210>  22
<211>  816
<212>  DNA
<213>  Artificial Sequence

<220>
<223>  nucleotide sequences of CTBTri4573 expression cassette

<400>  22
tctagatgca taattcgtgt cgctcaaggc gcactcccgt tctggataat gttttttgcg       60

ccgacatcat aacggttctg gcaaatattc tgaaatgagc tgttgacaat taatcatcgg      120

ctcgtataat gtgtggaatt gtgagcggat aacaatttca cacaggaaac agaattcatg      180

aagaaaattt ggctggcctt agccggcctg gttctggcat tcagcgccag cgcaaccccg      240

cagaacatca ccgacctgtg cgccgagtac cacaacaccc aaatttatac cctgaacgac      300

aaaattttta gctacaccga gagcctggca ggcaagcgcg agatggccat catcaccttc      360

aagaacggcg ccattttcca ggtggaggtg ccgggcagcc agcacatcga cagtcagaag      420

aaggccatcg agcgcatgaa ggacaccctg cgcatcgcct acctgaccga ggccaaggtg      480

gagaagctgt gcgtgtggaa caacaagacc ccgcacgcca tcgccgcaat cagcatggcc      540

aacggcggat ccggccgcct gctgctgcgt ctggaagaac tggaacgtcg cctggaagag      600

ctggcaaagt tcgtggcagc atggaccctg aaagcggctg cggttgacct ggaactggct      660

gctctgcgtc gtcgtctgga ggagctggct cgtggcggtt ctggtagcgc cgtgaccgag      720

tactatctga accatggcga gtggccgggt aataacacca gcgccggcgt ggccacaagc      780

agtgagatca agctcgagca ccaccaccac caccac                                816

<210>  23
<211>  198
<212>  PRT

<213>  Artificial Sequence

<220>
<223>  amino acid sequences of StxBTri4573

<400>  23

Met Lys Lys Ile Trp Leu Ala Leu Ala Gly Leu Val Leu Ala Phe Ser
1               5                   10                  15

Ala Ser Ala Lys Lys Thr Leu Leu Ile Ala Ala Ser Leu Ser Phe Phe
            20                  25                  30

Ser Ala Ser Ala Leu Ala Thr Pro Asp Cys Val Thr Gly Lys Val Glu
        35                  40                  45

Tyr Thr Lys Tyr Asn Asp Asp Asp Thr Phe Thr Val Lys Val Gly Asp
    50                  55                  60

Lys Glu Leu Phe Thr Asn Arg Trp Asn Leu Gln Ser Leu Leu Leu Ser
65                  70                  75                  80

Ala Gln Ile Thr Gly Met Thr Val Thr Ile Lys Thr Asn Ala Cys His
                85                  90                  95

Asn Gly Gly Gly Phe Ser Glu Val Ile Phe Arg Gly Gly Ser Gly Arg
            100                 105                 110

Leu Leu Leu Arg Leu Glu Glu Leu Glu Arg Arg Leu Glu Glu Leu Ala
        115                 120                 125

Lys Phe Val Ala Ala Trp Thr Leu Lys Ala Ala Ala Val Asp Leu Glu
    130                 135                 140

Leu Ala Ala Leu Arg Arg Arg Leu Glu Glu Leu Ala Arg Gly Gly Ser
145                 150                 155                 160

Gly Ser Ala Val Thr Glu Tyr Tyr Leu Asn His Gly Glu Trp Pro Gly
                165                 170                 175

Asn Asn Thr Ser Ala Gly Val Ala Thr Ser Ser Glu Ile Lys Leu Glu
            180                 185                 190

His His His His His His
        195

<210>  24
<211>  771
<212>  DNA
<213>  Artificial Sequence

<220>
<223>  nucleotide sequences of StxBTri4573 expression cassette

<400>  24
tctagatgca taattcgtgt cgctcaaggc gcactcccgt tctggataat gttttttgcg      60

ccgacatcat aacggttctg gcaaatattc tgaaatgagc tgttgacaat taatcatcgg     120

ctcgtataat gtgtggaatt gtgagcggat aacaatttca cacaggaaac agaattcatg     180

aagaaaattt ggctggcctt agccggcctg gttctggcat tcagcgccag cgcaaaaaaa     240

acattattaa tagctgcatc gctttcattt ttttcagcaa gtgcgctggc gacgcctgat     300

tgtgtaactg gaaaggtgga gtatacaaaa tataatgatg acgatacctt tacagttaaa     360

gtgggtgata aagaattatt taccaacaga tggaatcttc agtctcttct tctcagtgcg     420

caaattacgg ggatgactgt aaccattaaa actaatgcct gtcataatgg agggggattc     480

agcgaagtta tttttcgtgg cggatccggc cgcctgctgc tgcgtctgga agaactggaa     540

cgtcgcctgg aagagctggc aaagttcgtg gcagcatgga ccctgaaagc ggctgcggtt     600

gacctggaac tggctgctct gcgtcgtcgt ctggaggagc tggctcgtgg cggttctggt     660

agcgccgtga ccgagtacta tctgaaccat ggcgagtggc cgggtaataa caccagcgcc     720

ggcgtggcca caagcagtga gatcaagctc gagcaccacc accaccacca c              771

<210>  25
<211>  213
<212>  PRT
<213>  Artificial Sequence

<220>
<223>  amino acid sequences of LTBTri4573

<400>  25

Met Lys Lys Ile Trp Leu Ala Leu Ala Gly Leu Val Leu Ala Phe Ser
1               5                   10                  15

Ala Ser Ala Ala Pro Gln Thr Ile Thr Glu Leu Cys Ser Glu Tyr Arg
            20                  25                  30

Asn Thr Gln Ile Tyr Thr Ile Asn Asp Lys Ile Leu Ser Tyr Thr Glu
        35                  40                  45

Ser Met Ala Gly Lys Arg Glu Met Val Ile Ile Thr Phe Lys Ser Gly
    50                  55                  60

Glu Thr Phe Gln Val Glu Val Pro Gly Ser Gln His Ile Asp Ser Gln
65                  70                  75                  80

Lys Lys Ala Ile Glu Arg Met Lys Asp Thr Leu Arg Ile Thr Tyr Leu

85                  90                  95

Thr Glu Thr Lys Ile Asp Lys Leu Cys Val Trp Asn Asn Lys Thr Pro
            100                 105                 110

Asn Ser Ile Ala Ala Ile Ser Met Lys Asn Gly Gly Ser Gly Arg Leu
        115                 120                 125

Leu Leu Arg Leu Glu Glu Leu Glu Arg Arg Leu Glu Glu Leu Ala Lys
    130                 135                 140

Phe Val Ala Ala Trp Thr Leu Lys Ala Ala Ala Val Asp Leu Glu Leu
145                 150                 155                 160

Ala Ala Leu Arg Arg Arg Leu Glu Glu Leu Ala Arg Gly Gly Ser Gly
                165                 170                 175

Ser Ala Val Thr Glu Tyr Tyr Leu Asn His Gly Glu Trp Pro Gly Asn
            180                 185                 190

Asn Thr Ser Ala Gly Val Ala Thr Ser Ser Glu Ile Lys Leu Glu His
        195                 200                 205

His His His His His
    210

<210>  26
<211>  816
<212>  DNA
<213>  Artificial Sequence

<220>
<223>  nucleotide sequences of LTBTri4573 expression cassette

<400>  26
tctagatgca taattcgtgt cgctcaaggc gcactcccgt tctggataat gttttttgcg      60

ccgacatcat aacggttctg gcaaatattc tgaaatgagc tgttgacaat taatcatcgg      120

ctcgtataat gtgtggaatt gtgagcggat aacaatttca cacaggaaac agaattcatg      180

aagaaaattt ggctggcctt agccggcctg gttctggcat tcagcgccag cgcagcacct      240

cagaccatta ccgaactgtg cagtgaatat cgcaataccc agatctatac cattaatgat      300

aagattctga gctacaccga aagtatggcc ggtaaacgcg aaatggttat tattaccttt      360

aagagtggcg aaacctttca ggttgaagtt ccgggtagcc agcatattga tagtcagaaa      420

aaagcaattg agcgcatgaa agataccctg cgcattacct atctgaccga aaccaaaatt      480

gataaactgt gcgtttggaa taacaaaacc ccgaatagca ttgcagcaat tagtatgaaa      540

aacggcggat ccggccgcct gctgctgcgt ctggaagaac tggaacgtcg cctggaagag      600

ctggcaaagt tcgtggcagc atggaccctg aaagcggctg cggttgacct ggaactggct 660

gctctgcgtc gtcgtctgga ggagctggct cgtggcggtt ctggtagcgc cgtgaccgag 720

tactatctga accatggcga gtggccgggt aataacacca gcgccggcgt ggccacaagc 780

agtgagatca agctcgagca ccaccaccac caccac 816

<210> 27
<211> 250
<212> PRT
<213> Artificial Sequence

<220>
<223> amino acid sequences of SubBTri4573

<400> 27

Met Lys Lys Ile Trp Leu Ala Leu Ala Gly Leu Val Leu Ala Phe Ser
1               5                   10                  15

Ala Ser Ala Thr Ile Lys Arg Phe Phe Val Cys Ala Gly Ile Met Gly
            20                  25                  30

Cys Leu Ser Leu Asn Pro Ala Met Ala Glu Trp Thr Gly Asp Ala Arg
        35                  40                  45

Asp Gly Met Phe Ser Gly Val Val Ile Thr Gln Phe His Thr Gly Gln
    50                  55                  60

Ile Asp Asn Lys Pro Tyr Phe Cys Ile Glu Gly Lys Gln Ser Ala Gly
65                  70                  75                  80

Ser Ser Ile Ser Ala Cys Ser Met Lys Asn Ser Ser Val Trp Gly Ala
                85                  90                  95

Ser Phe Ser Thr Leu Tyr Asn Gln Ala Leu Tyr Phe Tyr Thr Thr Gly
            100                 105                 110

Gln Pro Val Arg Ile Tyr Tyr Glu Pro Gly Val Trp Thr Tyr Pro Pro
        115                 120                 125

Phe Val Lys Ala Leu Thr Ser Asn Ala Leu Val Gly Leu Ser Thr Cys
    130                 135                 140

Thr Thr Ser Thr Glu Cys Phe Gly Pro Asp Arg Lys Lys Asn Ser Gly
145                 150                 155                 160

Gly Ser Gly Arg Leu Leu Leu Arg Leu Glu Glu Leu Glu Arg Arg Leu
                165                 170                 175

Glu Glu Leu Ala Lys Phe Val Ala Ala Trp Thr Leu Lys Ala Ala Ala
            180                 185                 190

Val Asp Leu Glu Leu Ala Ala Leu Arg Arg Arg Leu Glu Glu Leu Ala
        195                 200                 205

Arg Gly Gly Ser Gly Ser Ala Val Thr Glu Tyr Tyr Leu Asn His Gly
    210                 215                 220

Glu Trp Pro Gly Asn Asn Thr Ser Ala Gly Val Ala Thr Ser Ser Glu
225                 230                 235                 240

Ile Lys Leu Glu His His His His His His
                245                 250

<210>  28
<211>  927
<212>  DNA
<213>  Artificial Sequence

<220>
<223>  nucleotide sequences of SubBTri4573 expression cassette

<400>  28
tctagatgca taattcgtgt cgctcaaggc gcactcccgt tctggataat gttttttgcg      60

ccgacatcat aacggttctg gcaaatattc tgaaatgagc tgttgacaat taatcatcgg     120

ctcgtataat gtgtggaatt gtgagcggat aacaatttca cacaggaaac agaattcatg     180

aagaaaattt ggctggcctt agccggcctg gttctggcat tcagcgccag cgcaaccatt     240

aagcgctttt tcgtgtgtgc aggcattatg ggttgtctga gcctgaatcc ggcaatggcc     300

gaatggaccg gcgatgcacg tgatggcatg tttagcggtg ttgtgattac ccagtttcat     360

accggtcaga ttgataataa gccgtatttt tgtatcgagg gcaaacagag tgccggtagt     420

agcattagtg catgtagcat gaaaaatagc agtgtgtggg gcgccagctt tagtaccctg     480

tataatcagg cactgtattt ttataccacc ggtcagccgg tgcgcatcta ttatgaaccg     540

ggcgtgtgga cctatccgcc gtttgtgaaa gcactgacca gtaatgccct ggttggcctg     600

agcacctgca ccaccagcac cgaatgtttt ggtccggatc gtaaaaagaa tagtggcgga     660

tccggccgcc tgctgctgcg tctggaagaa ctggaacgtc gcctggaaga gctggcaaag     720

ttcgtggcag catggaccct gaaagcggct gcggttgacc tggaactggc tgctctgcgt     780

cgtcgtctgg aggagctggc tcgtggcggt tctggtagcg ccgtgaccga gtactatctg     840

aaccatggcg agtggccggg taataacacc agcgccggcg tggccacaag cagtgagatc     900

aagctcgagc accaccacca ccaccac                                         927

<210>  29
<211>  29
<212>  PRT
<213>  Artificial Sequence

<220>
<223>  Glycosylation sequence £¨PilE S45~K73£©

<400>  29

Ser Ala Val Thr Glu Tyr Tyr Leu Asn His Gly Glu Trp Pro Gly Asn
1               5                   10                  15

Asn Thr Ser Ala Gly Val Ala Thr Ser Ser Glu Ile Lys
            20                  25

<210>  30
<211>  4
<212>  PRT
<213>  Artificial Sequence

<220>
<223>  Linker

<400>  30

Gly Gly Ser Gly
1

<210>  31
<211>  19
<212>  PRT
<213>  Artificial Sequence

<220>
<223>  DsbA signal peptide

<400>  31

Met Lys Lys Ile Trp Leu Ala Leu Ala Gly Leu Val Leu Ala Phe Ser
1               5                   10                  15

Ala Ser Ala

<210>  32
<211>  37
<212>  DNA
<213>  Artificial Sequence

<220>
<223>  Primer SacI-UP

<400>  32
cgagctctgc ataattcgtg tcgctcaagg cgcactc                              37

<210>  33

<211>  36
<212>  DNA
<213>  Artificial Sequence

<220>
<223>  Primer XhoI-Down

<400>  33
ccgctcgagc ttgatctctc actgcttgtg gccacg                                36